(19) Europäisches Patentamt
European Patent Office
Office européen des brevets

(11) EP 1 935 434 A1

(12) EUROPEAN PATENT APPLICATION

(43) Date of publication:
**25.06.2008 Bulletin 2008/26**

(51) Int Cl.:
***A61K 47/48*** *(2006.01)*

(21) Application number: **06077234.0**

(22) Date of filing: **19.12.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **novosom AG**
**06120 Halle (DE)**

(72) Inventor: **Panzner, Steffen**
**D-06114 Halle (DE)**

(74) Representative: **Crump, Julian Richard John et al**
**Mintz Levin Cohn Ferris Glovsky & Popeo Intellectual Property LLP**
**The Rectory**
**9 Ironmonger Lane**
**London EC2V 8EY (GB)**

Remarks:
Claims 11-12, 14-16, 19-23, 26-30, 34-35, 42-48, 53-68 are deemed to be abandoned due to non-payment of the claims fees (Rule 31 (2) EPC).

# (54) Construction and use of transfection enhancer elements

(57) This disclosure describes structural elements that enable transport of otherwise impermeable polar substances across biological membranes, in particular cell membranes. The elements are pH sensitive in terms of charge and hydrophilicity and undergo a polar - apolar transition when exposed to low pH.

EP 1 935 434 A1

## Description

### Field of the invention

This disclosure describes structural elements that enable transport of otherwise impermeable polar substances across biological membranes, in particular cell membranes. The elements are pH sensitive in terms of charge and hydrophilicity and undergo a polar - apolar transition when exposed to low pH.

### Background of the invention

Biological cells are surrounded and sealed with a continuous membrane which is impermeable to polar solutes irrespective of their molecular dimension. Although very small molecules such as water or urea are still able to penetrate a lipid bilayer, diffusion of charged ions is already very slow and the membrane is practically impermeable for somewhat bigger polar molecules, e.g. glucose or calcein. Independent from size, hydrophilicity is the second big factor that has an impact on the ability of a molecule to penetrate lipid bilayers and molecules showing some solubility in both the watery phase and the lipid phase can cross lipid bilayers and equilibrate between both sides of the membrane, e.g. the interior and exterior of a cell or the aqueous inner volume and the external buffer phase of a liposome. This basic understanding is guiding the synthesis and optimization of small molecule drugs and is part of the "Lipinski rule of five" (Lipinski et al., Advanced Drug Delivery Reviews, 23, 3-25, 1997). Hydrophilicity is expressed as logP, the equilibrium constant of a given solute between 1-octanol and water; values >1 indicate a preferential distribution into the apolar phase of 1-octanol, values <1 indicate better solubility in water than 1-octanol.
In many cases the molecules of interest comprise protonable groups of either acidic or basic character and the state of protonation has an impact on the hydrophilicity of the molecule. A common descriptor that reflects pH dependent hydrophilicity is logD, which is the logP at a given pH.
The knowledge for such changes in the distribution is widely used in chemical synthesis and purification, e.g. when a weakly basic molecule is re-distributed from a water phase into the organic phase of a two phase system by raising the pH of the solution.
More specifically, such understanding is fundamental for practicing the remote loading of liposomes with small pH-sensitive solutes as disclosed in Madden et al., Chem. Phys. Lipids, 53(1), 37-46, 1990 or Harrigan et al., Biochim. Biophys. Acta, 1149(2), 329-338, 1993, amongst others. Drugs like doxorubicin are provided in a buffer of neutral or slightly basic pH at or above the pka of the molecule. The unprotonated form is membrane permeable and equilibrates between the bulk solution and the interior of added liposomes. Now, if such liposomes contain a buffer of low pH, the drug molecule becomes protonated and cannot escape the liposome anymore. The process continues until the buffer capacity of the liposome interior is exhausted or all drug resides within the liposome and distributions far from equilibrium can be reached.
The same principle can be applied to weak acids, e.g. the carboxylic acids which are soluble or miscible with organic solvents in their undissociated form but not in the salt form.
Penetration of carboxylic acids through lipid bilayers has been reported e.g. by Clercs et al., Biochim. Biophys. Acta, 1240(2), 257-265, 1995.

### Objects of the invention

The invention disclosed here takes advantage of the pH-dependent hydrophilicity effect for the transport and distribution of polar molecules across membranes.
An object of the invention is therefore to provide pH-sensitive hydrophilicity elements, so called transfection enhancer elements or TEE's within this disclosure, capable of transporting polar molecules across membranes.
A particular object of the invention is to provide combinations of TEE's with nucleic acids.
Another object of the present invention is to provide combinations of one or more TEE's with macromolecules.
Another object of the invention is to provide combinations of TEE's with peptides or proteins.
A particular object of the invention is to provide a combinations of TEE's with carrier systems sequestering the active ingredients.
Yet another object of the present invention is to provide combinations of TEE's with small molecules, active pharmaceutical ingredients, sugars or other polar molecules from different origin.

### Brief description of the invention

According to one aspect of the present invention therefore there are provided conjugates of nucleic acids with two or more pH-responsive transfection enhancer elements (TEE's) with the general structure (I)

Hydrophobic element - pH-responsive hydrophilic elements (I)

The position of the hydrophilic element within the TEE structure may vary. In some aspects, the hydrophilic element is located distal from the link between molecule and TEE. In other aspects, the hydrophilic element is located central within the TEE.

In some embodiments said pH-responsive hydrophilic element comprises weak acids having a pka of between 2 and 6, preferred of between 3 and 5. Said weak acids may be selected from carboxyl groups, barbituric acid and derivatives thereof, xanthine and derivatives thereof, wherein in some embodiments the xanthine derivatives are pyrimidines.

In other embodiments said pH-responsive hydrophilic element may be a zwitterionic structure comprising a combination of weak or strong acidic groups with weak bases, the latter having a pka of between 3 and 8, preferred of between 4.5 and 7. Suitably said zwitterionic structures may be formed from an anionic group and a heterocyclic nitrogen atom as cationic group.

To achieve specific pKa's of said hydrophilic elements in one aspect of the invention said pH-responsive hydrophilic element may be substituted with structural elements, selected from the group comprising hydroxymethyl-, hydroxyethyl-, methoxymethyl-, methoxyethyl-, ethoxymethyl-, ethoxyethyl-, thiomethyl-, thioethyl-, methylthiomethyl-, methylthioethyl-, ethylthiomethyl-, ethylthioethyl-, chlorid-, chlormethyl- vinyl-, phenyl-, benzyl-, methyl-, ethyl- , propyl-, isopropyl- and tert-butyl or cyclohexyl groups.

In some embodiments said hydrophobic element comprise linear, branched or cyclic chains with a minimum chain length of 6 elements. In one aspect of this embodiment said hydrophobic element comprises more than 6 and up to 40 elements, in a second aspect said hydrophobic element comprises between 6 and 20 elements and in a third aspect said hydrophobic element comprises between 20 and 40 elements.

The chain elements of said hydrophobic element may be carbon atoms. In one embodiment said hydrophobic element can be saturated or may contain unsaturated bonds. In other embodiments said hydrophobic element may be substituted.

In some embodiments the branching of the main chain of said hydrophobic element may comprise rather small building blocks. Preferred building blocks comprise methyl-, ethyl-, propyl-, isopropyl-, methoxy-, ethoxy-, methoxymethyl-, ethoxymethyl-, methoxyethyl-, ethoxyethyl- and vinyl- or halogen groups or mixtures thereof.
Alternatively, said hydrophobic element may derive from sterols, said sterols may be further substituted.

It is possible to insert one or more heteroatoms or chemical groups into the hydrophobic element of the pH-responsive transfection enhancer elements (TEE's). Such heteroatoms or chemical groups may be selected from -O-, -S-, -N(H)C(O)-, -C(O)O-, -OC(O)N(H)-, -C(O)-, -C(O)-N(H)-, -N(H)-C(O)-O-, - CH=N-, -O-C(O)-, -N=CH- and/or -S-S-, amino acids or derivatives thereof, $\alpha$-hydroxyacids or $\beta$-hydroxy acids.

TEE's undergo a hydrophile-hydrophobe transition in response to an acidification of the environment. This transition is mediated by the hydrophilic elements described above that are responsive towards pH.

In preferred embodiments of the invention logD(4.0)-logD(7.4)>=1 for the transfection enhancer elements and logD at pH 7,4 is between -2 and 10.

In other embodiments polymers such as nucleic acids, oligonucleotides or polynucleotides are described, wherein polymer fragments or monomers of said polymer are modified with one or more TEE's. In preferred embodiments, the sum of logD(polymer fragment)+logD(TEE's)> -10; more preferred this is >-5 and logD is determined for pH7.4.

In most aspects of the invention, the logD at pH 4 of said pH-responsive transfection enhancer elements (TEE's) exceeds 0.

Of course, said pH-responsive transfection enhancer elements (TEE's) may contain more than one pH responsive hydrophilic element.

In one particular aspect said nucleic acid may be an oligonucleotide and the pH-responsive transfection enhancer elements (TEE's) are grafted onto said oligonucleotides via covalent chemical bonds. Such graft may sit at various positions including the 2'O- or 2'S-positions of the nucleosides, the nucleobase, the internucleoside linkages, e.g. the phosphate backbone or modifications thereof, the peptide backbone in peptide nucleic acids, the heterocyclic backbone in morpholino nucleic acids and the like. The graft may also be attached to the phosphoribose or phosphodexyribose backbone, replacing the former nucleobases at Cl.

In some embodiments no more than 2/3 or no more than 1/3 of all nucleobases of said oligonucleotide are modified with said pH-responsive transfection enhancer elements (TEE's).

In other embodiments only nucleobases at the flanks of said oligonucleotides are modified with pH-responsive transfection enhancer elements (TEE's)leading to gapmer structures.

In other preferred aspects, said oligonucleotide comprise polymeric or oligomeric extensions wherein the polymer backbone structure is not a phosphoribose and said extensions carry one or more TEE's. In some aspects such extension represents peptides or polyesters forming oligomeric or multimeric forms of TEE's, such as TEE's linked to

peptides, α- or β-amino acids or α- or β-hydroxyacids.

Said pH-responsive transfection enhancer elements (TEE's) may be alkylcarboxylic acids preferably comprising between 8 to 16 carbon atoms. Alternatively, said pH-responsive transfection enhancer elements (TEE's) may be sterols.

It is also possible that said pH-responsive transfection enhancer elements are grafted onto polycationic elements that form a precipitate, a complex controlled in size or a stoichiometric associate with said nucleic acids.

In some embodiments said polycationic elements are polyamines selected from the group comprising polyethyleneimine, spermine, thermine, spermidine, putrescine, polylysine or polyarginine and derivatives thereof or said polycationic elements are lipopolyamines selected from the group comprising cholesteryl polyamine carbamates, DOSPER, DOGS or DOSPA.

In another particular embodiment of the present invention, said nucleic acids are oligonucleotides, polynucleotides or DNA plasmids.

In yet another aspect of the present invention there is provided a pharmaceutical composition comprising conjugates of nucleic acids with transfection enhancer elements (TEE's) in accordance with the present invention and a pharmaceutically acceptable vehicle therefor.

In yet another aspect, the present invention comprehends the use of a pharmaceutical composition according to the present invention for the treatment or prophylaxis of inflammatory, immune or autoimmune disorders and/or cancer of humans or non-human animals.

In another aspect, the present invention comprehends the use of pH responsive transfection enhancer elements (TEE's) for the in vivo, in vitro or ex vivo transfection of polar solutes.

In some embodiments said polar solutes may be selected from the group comprising small molecules, proteins, peptides, carbohydrates or nucleic acids.

One or more transfection enhancer elements (TEE's) may be conjugated to said polar solutes by chemical bonds, physical attraction or by other interactions.

Alternatively, said transfection enhancer elements (TEE's) may be conjugated to carrier systems sequestering said polar solutes.

For clarity, the following definitions and understandings are used for important terms of the invention:

LogP

...is the ratio of the respective concentrations of a compound in the 1-octanol and water partitions of a two-phase system at equilibrium. The octanol-water partition coefficient (logP) is used to describe the lipophilic or hydrophobic properties of a compound.

LogD

...is the ratio of the equilibrium concentrations of all species (unionized and ionized) of a molecule in 1-octanol to same species in the water phase. The partition coefficient for dissociative mixtures, logD, is defined as follows:

$$\log D = \log\ (\Sigma(c_i^{H2O})/\Sigma(c_i^{org})),$$

where

$C_i^{H2O}$ is the concentration of the i-th microspecies in water and
$C_i^{org}$ is the concentration of the i-th microspecies in the organic phase.

LogD differs from logP in that ionized species are considered as well as the neutral form of the molecule. LogD is therefore the logP at a given pH of the medium.

LogP and logD values can be determined experimentally by measuring the partition of a molecule or its ionized forms in octanol/water systems. Experimental values have been generated for a vast amount of individual compounds and expert systems allow extrapolating logP and logD values for novel species. One such expert system is ACD/Labs with the modules ACD/LogP or ACD/logD and ACD/Labs 7.06 has been used for calculations within this disclosure.

"Nucleic acid" or "Polynucleotide"

.. as used herein refers to any nucleic acid containing molecule, including without limitation, DNA or RNA. The term polynucleotide(s) generally refers to any polyribonucleotide or polydeoxyribonucleotide, which may be unmodified RNA or DNA or modified RNA or DNA. Thus, for instance, polynucleotides as used herein refers to, among others, single-and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions.

Oligonucleotide

as used herein is defined as a molecule with two or more deoxyribonucleotides or ribonucleotides, often more than three, and usually more than ten. The exact size of an oligonucleotide will depend on many factors, including the ultimate function or use of the oligonucleotide. Oligonucleotides can be prepared by any suitable method, including, for example, cloning and restriction of appropriate sequences and direct chemical synthesis by a method such as the phosphotriester method of Narang et al., Meth. Enzymol., 68, 90-99, 1979; the phosphodiester method of Brown et al., Method Enzymol., 68, 109-151, 1979 the diethylphosphoramidite method of Beaucage et al., Tetrahedron Lett., 22, 1859-1862, 1981 the triester method of Matteucci et al., J. Am. Chem. Soc., 103, 3185-3191, 1981 or automated synthesis methods; and the solid support method of US 4,458,066.

Transfection

...is used widely to specifically describe the disappearance of a concentration gradient across a biological membrane in vivo, in vitro or ex-vivo. It comprises transport across, or diffusion through, penetration or permeation of biological membranes irrespective of the actual mechanism by which said processes occur.

**Detailed description of the invention**

Transfection enhancer elements are pH-responsive amphiphiles with the general structure:

(I) hydrophobic element - pH-responsive hydrophilic element

The pH-sensitive hydrophilicity elements are called transfection enhancer elements or TEE's within this disclosure.

Hydrophilic elements of the TEE's

In one aspect of this invention the hydrophilic elements are weak acids that provide a response in hydrophilicity between pH values of about 4 and the physiological pH of 7.4.
Carboxyl groups, barbituric acid or derivatives thereof, in particular xanthine or derivatives thereof or pyrimidines and derivatives therof of formula (1) to (3) in table 1 represent, but do not limit such pH-responsive hydrophilic elements.

Table 1: Compounds 1-3

| | |
|---|---|
| (1) 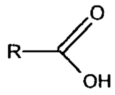 | (1) Carboxylic acids. R represents the hydrophobic element of the invention. |
| 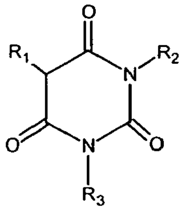 (2) | (2) Barbituric acid derivatives. R1, R2 or R3 may represent the hydrophobic element of the invention. |
| (3) 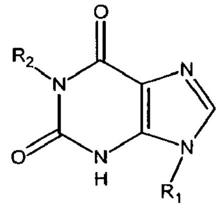 | (3) Xanthine derivatives. R1 or R2 represent the hydrophobic element of the invention. |

LogD values for hydrophilic head groups derived from (1) to (3) are high at low pH and low at neutral or higher pH.
In another aspect of the invention, the hydrophilic elements comprise zwitterionic groups that respond to changes in the pH of the environment.
Zwitterionic structures exist at pH values where both the cationic and the anionic group are charged and a generalized logD plot is shown in figure 1. It is apparent that the zwitterions have higher logD values than the charged parent groups and the maximum amplitude in logD for the zwitterion formation is about 2.5 units.
The desired increase in logD upon acidification is represented by the right flank of the logD curve and depends on the pKa of the cationic charge group; it is rather independent from the pKa of the anionic group itself. As an example, the anionic group maybe a carboxyl group and the cationic group maybe a heterocyclic nitrogen atom two to five carbon atoms apart from that group (e.g. compounds 10 or 11). Pyridylcarboxylic acids, imidazolcarboxylic acids or the like are a few representations of such pH-responsive hydrophilic elements. The zwitterion exists between pH 4 and pH 7, thereby providing the pH-responsive hydrophilic headgroups of the invention. On the contrary, a simple amino group having a high pKa of about 9 (e.g. compound 13) or a quarternary ammonium group providing a constant positive charge with no effective pKa (e.g. compound 12) extends the range of pH values where the zwitterion exists and any change in hydrophilicity does no longer occur in the pH region desired (pH 2 to 9 or the more preferred ranges given above, see figure 1 and table 2). It becomes apparent that the guiding pKa for the zwitterion is the pKa of the basic counterpart. In preferred embodiments, the pKa for a base in zwitterions is between 3 and 8, preferred between 4.5 and 7 to achieve a pH dependent response in logD between pH 4 and 7.4.

Table 2: Compounds 10-14

| | | |
|---|---|---|
| (10) 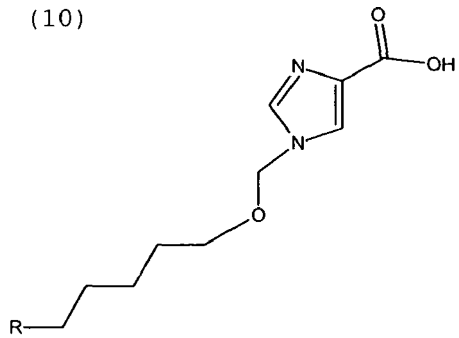 | (12) 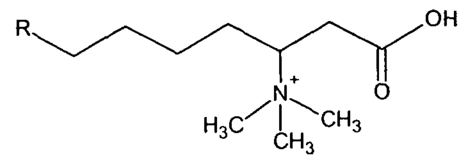 | (11) 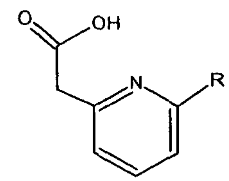 |
| (13) 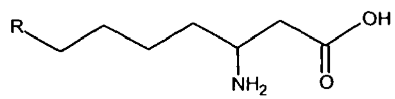 | (14) 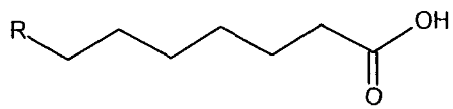 | |

The hydrophilic elements can further be substituted with polar or apolar groups. In one aspect of the invention, substitutions are selected to achieve a specific pKa of the hydrophilic element. Rules to achieve such adjustment of pKa values are known to the skilled artisan and comprise for example substitutions at nitrogen atoms of barbituric acid or xanthine with hydroxymethyl-, hydroxyethyl-, methoxymethyl-, methoxyethyl-, ethoxymethyl-, ethoxyethyl-, thiomethyl-, thioethyl-, methylthiomethyl-, methylthioethyl-, ethylthiomethyl-, ethylthioethyl-, chlorid-, chlormethyl-vinyl-, phenyl- or benzyl groups or mixtures thereof to achieve a lower pK of the structure. Substitutions at the positions R1, R2 or R3 in formula (2) or (3) are in particular suitable to achieve such shift in pK values. Of course, pK values can be shifted towards higher values with substitutents comprising methyl-, ethyl- , propyl-, isopropyl- and tert-butyl or cyclohexyl groups or mixtures therof.
It is known, that the pKa value for carboxyl groups is also affected by substitutions or chemical alterations in spatial proximity. Acrylic acid derivatives, aromatic carboxylic acids such as benzoic acid, pyridinyl carboxylic acid, α- or β-hydroxycarboxylic acids or α- or β-thiocarboxylic acids but also halogenated carboxylic acids have lower pKa values than the parent compounds. In contrast, substitutions with an +I effect change the pKa of a carboxyl group towards higher values; e.g. in cyclohexylcarboxylic acids.
Specific examples of substituted hydrophilic elements include, but are not limited to formula (4) to (9) of table 3, wherein R identifies the hydrophobic element of the TEE:

Table 3: Compounds 4-9

| Substituted xanthins | | |
|---|---|---|
| (4) 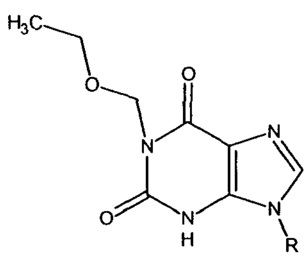 | (5) 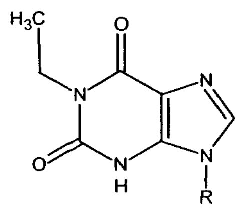 | (6) 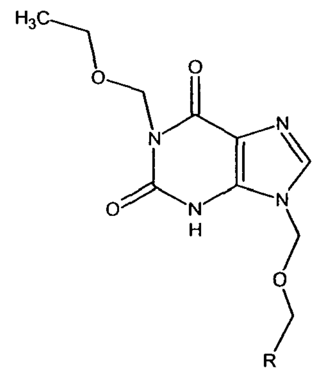 |
| Substituted carboxylic acids | | |
| (7) 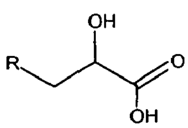 | (8) 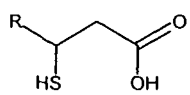 | 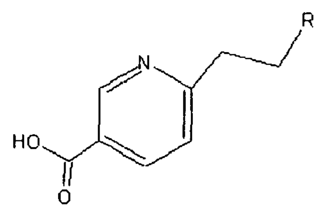 (9) |

Other derivatives of xanthins, pyrimidins (uracils) or barbituric acids are disclosed below and analyzed with respect to their logD values at pH4.0 and pH7.4. The methoxyethyl moeity in compounds (100) to (129) represents or may be replaced by the hydrophobic elements of the TEE as described above.

| Chemical structure | Compound-# | logD (pH4) | logD (pH 7.4) |
|---|---|---|---|
| 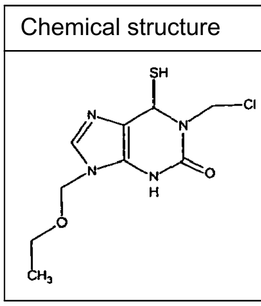 | (100) | -0,11 | -1,03 |

(continued)

| Chemical structure | Compound-# | logD (pH4) | logD (pH 7.4) |
|---|---|---|---|
| 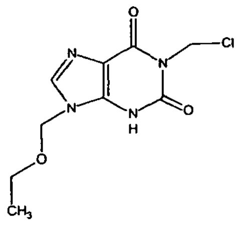 | (101) | -0,39 | -2, 18 |
| 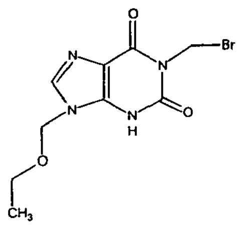 | (102) | 0,08 | -1,71 |
| 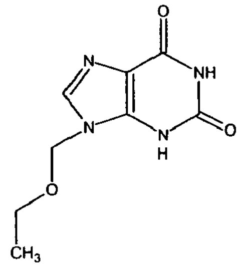 | (103) | -0,85 | -2,13 |
| 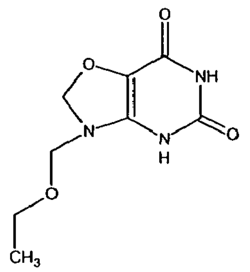 | (104) | -0,27 | -0,62 |
| 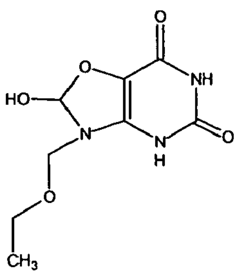 | (105) | -0, 18 | -0,77 |
| 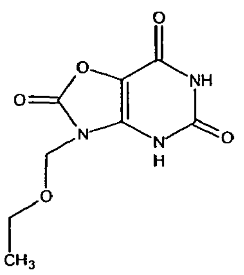 | (106) | -2,76 | -3,44 |

(continued)

| Chemical structure | Compound-# | logD (pH4) | logD (pH 7.4) |
|---|---|---|---|
| 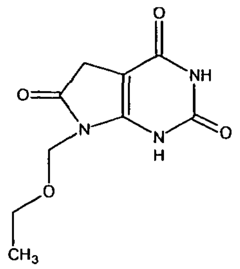 | (107) | -0,1 | -1,88 |
| 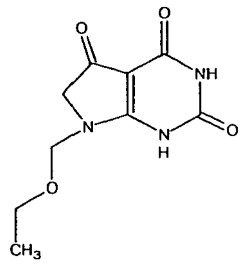 | (108) | -0,93 | -1,27 |
| 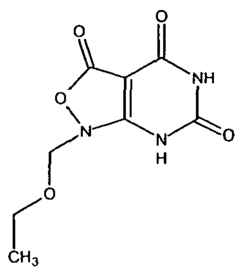 | (109) | -2,01 | -3,43 |
| 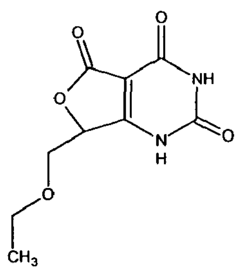 | (110) | -1,34 | -2,16 |
| 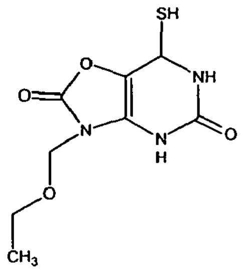 | (111) | 0,92 | -1,31 |

(continued)

| Chemical structure | Compound-# | logD (pH4) | logD (pH 7.4) |
|---|---|---|---|
| 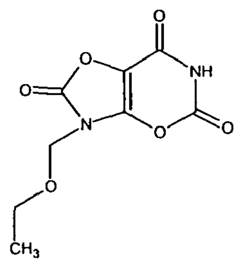 | (112) | -1,34 | -3,15 |
| 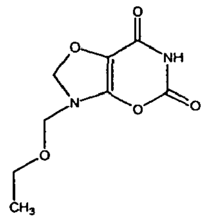 | (113) | -0,39 | -1,69 |
| 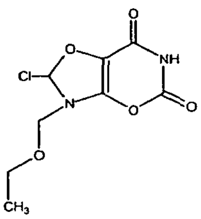 | (114) | 0,21 | -1,51 |
| 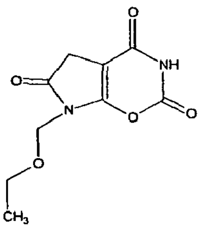 | (115) | 0,22 | -0,27 |
| 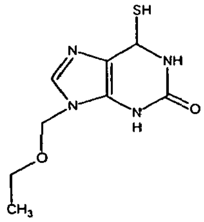 | (116) | -0,33 | -0,74 |
| 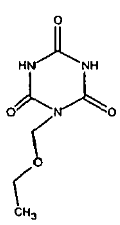 | (117) | -2,07 | -3,44 |

(continued)

| Chemical structure | Compound-# | logD (pH4) | logD (pH 7.4) |
|---|---|---|---|
| 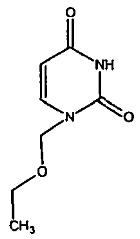 | (118) | -0,65 | -0, 68 |
| 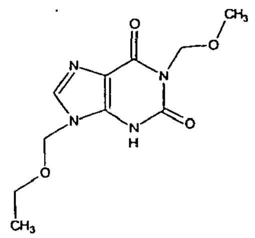 | (119) | -0,76 | -2,42 |
| 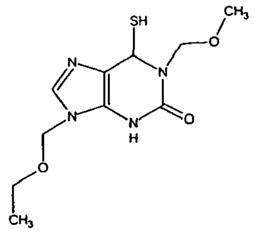 | (120) | -0,31 | -1,07 |
| 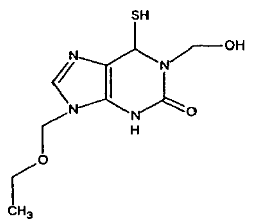 | (121) | -0,62 | -1,38 |
| 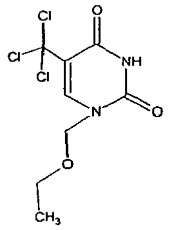 | (122) | 1,65 | 0,71 |
| 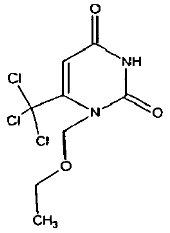 | (123) | 1,87 | 0,41 |

(continued)

| Chemical structure | Compound-# | logD (pH4) | logD (pH 7.4) |
|---|---|---|---|
| 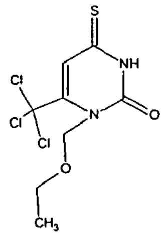 | (124) | 3,25 | 1,46 |
| 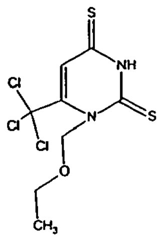 | (125) | 2,81 | 1,15 |
| 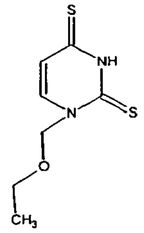 | (126) | 0,56 | -1,25 |
| 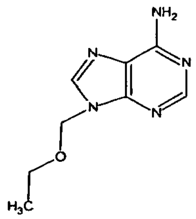 | (127) | -0,59 | -0,23 |
| 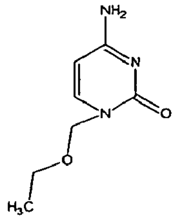 | (128) | -1,21 | -0,98 |
| 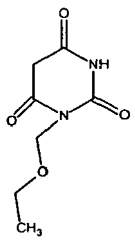 | (129) | -0,48 | -3,23 |

Further chemical representations for the hydrophilic elements can be identified from the group of weak acids using the relationship between logD, pH and the pKa of the substance. For acids, this can be expressed as follows:

$$\log D = \log P + \log(1 + 10^{(pH-pKa)});$$

wherein logP is the partition coefficient for the non-ionized form. The equation reflects conditions of zero ionic strenght and extremely low values for logD are calculated for acids at high pH. Under physiological conditions, where the ionic strenght is about 0,15M, salt formation is limiting such extremes in logD.

Figure 2 shows the logD calculations for a number of hydrophilic elements.

Further analysis reveals identical shifts in logD when curves are plotted against pH-pKa (see figure 3).

Once standardized with respect to their pKa values the logD plots become similar for all hydrophilic elements analyzed here. A maximum difference of 3.75 units in logD can be achieved for the ionization of a single hydrophilic element. The maximum amplitude for zwitterion formation is about 2.5 units in logD.

The full amplitude requires a rather large shift of about 6 units in pH. Within the practical range of ΔpH ~ 3.4 (pH 7.4 - pH 4) considered for many aspects of this invention, the maximum difference in logD is about 3 units for pKa ~ 4. The logD reacts very sensitive whenever the pH is 0 to 4 units above the pKa, the most sensitive reaction is at pH values between 1 and 2.5 units above pKa. This relation is also analyzed in the figure 4.

In practical terms, the ideal pKa for hydrophilic elements is about 4. Preferred are hydrophilic elements having a pKa between 2 and 6 (maximum amplitude about 1.5 units), more preferred are hydrophilic elements having a pKa between 3 and 5 (maximum amplitude about 2.5 units). Other hydrophilic elements within the scope of the invention may have pKa values between 1 and 7.

Hydrophobic elements of the TEE's

TEE's of this invention comprise hydrophobic elements which contribute to the penetration of biological lipid membranes. Chemical representations of such hydrophobic elements include linear, branched or cyclic chains with a minimum chain length of 6 elements. In many aspects of the invention the chain elements are carbon atoms. In some aspects of the invention the chain elements may comprise heteroatoms being able to form covalent bonds with more than one other chain element. Hydrogen or halogen atoms can substitute the chain, but are not elements of the chain. The hydrophobic elements can comprise more than 6 elements and may comprise up to 40 elements. In some aspects of the invention the hydrophobic elements comprise between 6 and 12 elements. In other aspects of the invention the hydrophobic element comprise between 12 and 20 elements. In still other aspects of the invention the hydrophobic element does comprise between 20 and 40 elements. In one aspect, the branching of the main chain comprises one or more rather small building blocks such as methyl-, ethyl-, propyl-, isopropyl-, methoxy-, ethoxy-, methoxymethyl-, ethoxymethyl-, methoxyethyl-, ethoxyethyl- and vinyl- or halogen groups or mixtures thereof.

Hydrophobic elements can be saturated or may contain unsaturated bonds. In another aspect, more complex branched and or cyclic ring systems may be chemical representations of the hydrophobic element. In one embodiment of such aspect, hydrophobic elements are derived from sterols. Of course, the sterols may further be substituted with methyl-, ethyl-, propyl-, isopropyl-, methoxy-, ethoxy-, methoxymethyl-, ethoxymethyl-, methoxyethyl-, ethoxyethyl- and vinyl- or halogen groups or mixtures thereof. In another aspect of the invention, the hydrophobic elements may comprise sterols that are substituted with one or more hydrophilic groups such as hydroxyl groups. In a preferred embodiment, sterols contain hydroxyl groups at one or more of the positions 3, 7 and 12. In another aspect, the sterol is a cholestan and in a preferred embodiment the cholestan is hydroxylated in one or more of the positions 3, 7 or 12.

It is possible to insert one or more heteroatoms or chemical groups into the hydrophobic element. In one embodiment of the invention said hydrophobic element comprises no more than 5 and in another embodiment no more than 2 heteroatoms or chemical groups. The heteroatoms or chemical groups may be selected from the group comprising -O-, -S-, -N(H)C(O)-, -C(O)O-, -OC(O)N(H)-, -C(O)-, -C(O)-N(H)-, -N(H)-C(O)-O-, -CH=N-, -O-C(O)-, -N=CH- and/or -S-S-.

Alternatively, said heteroatoms and/or chemical groups derive from amino acids, α-hydroxyacids or β-hydroxy acids.

In one embodiment said amino acid building block is selected from the group of proline, glycin, alanine, leucine, isoleucine, valin, tyrosine, tryptophane, phenylalanine or methionine or peptides thereof and said α-and β-hydroxyacids are selected from the group comprising glycolic acid, lactic acid or hydroxybutyric acid.

In another embodiment of such an aspect, more than a single ether group is present and the spacing between the ether bonds is two carbon atoms, representing the monomer elements in poly-ethylenglycol or polypropylenglycol.

Architecture of TEE's

Construction of TEE's is governed by its physicochemical parameters; TEE's undergo a hydrophile-hydrophobe

transition in response to an acidification of the environment. This transition is mediated by the hydrophilic elements described above that are responsive towards pH. Such response should have minimum amplitude of 0.5 logD whereby the higher absolute value of logD is achieved at the lower pH. In one aspect, such amplitude is higher than 1 logD which means that a distribution of such TEE from the water phase to the hydrophobic interior of the membrane becomes 10 times more preferred. In another aspect, such amplitude is higher than 1.5 and in some aspects the amplitude between the hydrophilic and hydrophobic form is more than 2 logD units.

In one aspect of the invention, the hydrophilic elements respond to pH values between the physiological pH and slightly acidic conditions of about pH 4. Such slightly acidic conditions can be found within cell organelles like endosomes or lysosomes. Therefore TEE's may be capable of mediating the endosomal escape of drugs after endocytotic uptake into the cell. Tumor tissue or areas of ongoing inflammation also provide a slightly acidic environment and consequently TEE's may be useful to accumulate drugs in these areas. Accumulation may occur specifically in tumor or stroma cells or in cells of the immune system or fibroblasts that are present in inflammatory regions. In a preferred embodiments the pK of the TEE is be optimized to maximize the difference between hydrophilicity at pH7.4 and hydrophobicity at pH4. In preferred aspects, the pka of the hydrophilic elements essentially driven by weak acids is between 2 and 6. In more preferred aspects this pka is between 3 and 5. As described above, the governing pka for the shift in hydrophilictiy can be the pka of a weak acidic group such as carboxylic acids, barbituric acids or xanthins. In cases where a shift in hyrophilicity is caused by zwitterion formation, the governing pka is the pka of a weak base such as pyridin, imidazol, morpholine or piperazine.

TEE's may contain one or more hydrophilic elements and the relative and absolute positioning of the hydrophilic elements may vary. In some cases, neighbouring effects may occur. Effects within the hydrophilic groups include, amongst others, pK shifts and zwitterion formation. Effects between hydrophilic groups may also include shifts in the respective pK values. This is known to the skilled artisan and frequently observed between carboxylic acids in close proximity, e.g. when the spacing between groups is between 2 to 5 carbon atoms.

Some examples for more complex hydrophilic elements are shown below (table 4).

Table 4: Compounds 15 and 16

| | |
|---|---|
| (15) 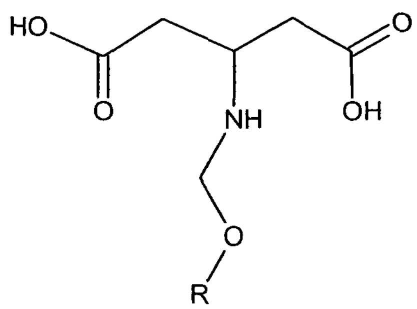 | Compound (15) β-Glutamic acid derivatives. R represents the hydrophobic element of the invention. The ether bond between R and the hydrophilic element is optional and lowers the pka of the amino group. |
| (16) 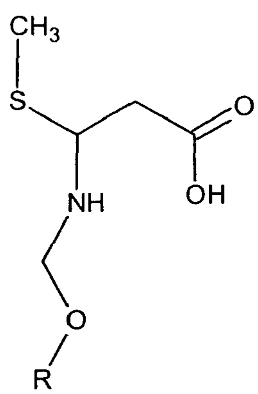 | Compound (16) 3-Amino-3-(methylthio)propanoic acid derivatives. R represents the hydrophobic element of the invention. Again, the ether bond between R and the amino group is optional but shifts the pka downwards and the same holds true for the thioether. The hydrophobic element may also be bound other positions including the methyl group at the thioether. |

TEE's with more than one hydrophilic element have larger amplitudes of hydrophilicity between their neutral and slightly acidic state. Of course, mixtures of hydrophilic elements can be combined with a single hydrophobic element. Such mixture may allow more precise adjustments in the amplitude and pH-sensitivity of the hydrophobic shift. However, too large of a number of hydrophilic elements increases the hydrophilicity of the TEE to values that can no longer be compensated with the hydrophobic shift.

Therefore, besides the amplitude of hydrophilicity between different pH values, the absolute hydrophilicity of the TEE at a first pH represents a very important aspect of the invention.

In terms of absolute hydrophilicity, the log D of the TEE itself may be vary between slightly hydrophilic at conditions of neutral or physiological pH and somewhat hydrophobic. In other words the TEE's have a logD at pH7.4 between -2 and 10. In some aspects, the logD (7.4) is greater than 1 and in some aspects the logD(7.4) greater than 3. In other aspects of the invention, the logD(7.4) of the TEE is smaller than 10, in some aspects the logD(7.4) is smaller than 7. In another aspect of the invention the logD of the TEE correlates with the logP or logD of the substance to be transported. In one variant of such aspect, the logD(7.4) of the TEE compensates logP or logD of the drug to be transported to an extent that the sum of both is bigger than -10. In preferred aspects, the sum is bigger than -5 and in even more preferred aspects the sum is bigger than -3. This combination alone may render the drug more amphipathic, thus improving its availability of transfection of cells. The additional contribution from the pH-sensitive element of the TEE will then facilitate a pH dependent transfection, e.g. from the endosomal pathway or at bodily sites of low pH such as in cancers or at sites of inflammation.

In yet another aspect of the invention, one or more TEE's may facilitate the transfection of a polymer, e.g. an oligonucleotide, polynucleotide, peptide or protein. The teachings from the previous paragraph can also be applied in such a case and the repetitive elements of the polymers (e.g. one or few amino acids or one or a few nucleotides) can be considered the drugs to be transported. As such, the TEE's chosen for such applications may have logD values close to the logP or log D constants of the repetitive element in question. Alternatively, averaging effects for polymers may allow selecting a smaller number of TEE's with higher logD.

Independent from its absolute logD at pH7.4, the logD of the TEE at pH4.0 needs to exceed 0.
TEE's with a negative logD(7.4) require high amplitudes of the hydrophobic shift and such high amplitudes can be provided hydrophilic elements comprising one or more carboxyl groups, xanthine groups or barbituric acid groups.

Specific examples of preferred TEE's:
The following chemical representations of TEE's should further illustrate the teachings of the invention. However, the scope of the invention is by no means limited to the specific examples given below. Preferred TEE's have the following attributes:

| | |
|---|---|
| Number of chain elements in the hydrophobic element | : 6...40 |
| logD(7.4) | : -2...10 |
| (correlates to logP of the drug to be transported) | |
| logD(7,4)-logD(4) | : >1 |
| pKa - | : 2 ... 6 |
| for weak acids | |
| for weak bases with ability for zwitterion formations | : 4.5 ... 7 |

A. TEE's based on carboxylic acids

In one aspect of the invention the TEE comprises one or more carboxylic acid groups as the hydrophilic element. In some embodiments of such aspect, the hydrophobic element comprises a straight chain of carbon atoms. In some representations, such chain is a straight alkyl chain.
Table 5 below is analyzing logD at pH4 and pH7.4 for different chain length of the carboxylic acids.

Table 5:

| # of C | pH 4,0 | pH 7,4 | Δ |
|---|---|---|---|
| 4 | 0,71 | -1,83 | -2,54 |
| 6 | 1,77 | -0,75 | -2,52 |
| 8 | 2,84 | 0,31 | -2,53 |
| 10 | 3,9 | 1,38 | -2,52 |
| 12 | 4,96 | 2,44 | -2,52 |
| 14 | 6,02 | 3,5 | -2,52 |
| 16 | 7,09 | 4,56 | -2,53 |
| 18 | 8,15 | 5,62 | -2,53 |
| 20 | 9,21 | 6,69 | -2,52 |

It becomes apparent that chain elongation by an methylene group increases the logD by about 0.5 units. Carboxylic acids with 6 to 26 C atoms represent preferred TEE's according to the selection criteria given above. Position effects of the carboxylic acid group are less important and the carboxylic group is not mandatory the terminal group of the hydrophobic element.

In some aspects, one or more positions of the main chain of the hydrophobic element can be substituted (R-) and the impact of some substitutions is analyzed below for hexadecanoic acid (palmitic acid) derivatives (table 6).

Table 6:

| methyl side chain | | | | ethyl side chain | | | | propyl side chain | | | |
|---|---|---|---|---|---|---|---|---|---|---|---|
| #subs | pH 4,0 | pH7,4 | ☐ | #subs | pH 4,0 | pH7,4 | ☐ | #subs | pH 4,0 | pH 7,4 | ☐ |
| 0 | 7,09 | 4,56 | -2,53 | 0 | 7,09 | 4,56 | -2.53 | 0 | 7,09 | 4,56 | -2,53 |
| 1 | 7,43 | 4,91 | -2,52 | 1 | 7,96 | 5.44 | -2,52 | 1 | 8,5 | 5,98 | -2,52 |
| 2 | 7,78 | 5,26 | -2,52 | 2 | 8,84 | 6,32 | -2,52 | 2 | 9,91 | 7,38 | -2,53 |
| 3 | 8,13 | 5,6 | -2,53 | 3 | 9.72 | 7,2 | -2,52 | 3 | 11,32 | 8,79 | -2,53 |

If R= methyl, each R results in a gain in logD of about 0.35 units. If R= ethyl, such gain is about 0.88 and for R=propyl the gain is about 1.41 per substitution. It becomes apparent that addition of methylene groups in side chain also increase logD by about 0.5 units as it is the case in the main chain.

In other aspects, R comprises heteroatoms, in particular oxygen atoms and the impact for some substitutions is analyzed below for hexadecanoic acid (palmitic acid) derivatives (table 7).

Table 7:

| methoxy side chain | | | | ethoxy side chain | | | | MOE side chain | | | |
|---|---|---|---|---|---|---|---|---|---|---|---|
| # subs | pH4.0 | pH7,4 | ☐ | # subs | pH 4,0 | pH7,4 | ☐ | #subs | pH 4,0 | pH7,4 | ☐ |
| 0 | 7,09 | 4,56 | -2,53 | 0 | 7,09 | 4,56 | -2.53 | 0 | 7,09 | 4,56 | -2,53 |
| 1 | 6,03 | 3,35 | -2,68 | 1 | 6,56 | 3,9 | -2,66 | 1 | 6,89 | 4,31 | -2,58 |
| 2 | 4,48 | 1,8 | -2,68 | 2 | 5.54 | 2,87 | -2,67 | 2 | 6,4 | 3,81 | -2,59 |
| 3 | 2,92 | 0,24 | -2,68 | 3 | 4,52 | 1,85 | -2,67 | 3 | 5,91 | 3,32 | -2,59 |

For R= methoxy each R results in a decrease of logD of about -1.4 units. If R= ethoxy, the extra methylene group contributes about 0,5 units of logD and the resulting effect is about -0,9 units of logD. If R= methoxyethyl, the average impact per substitution is about -0,4 units of logD.

Other substitutions on the side chain may further change the logD of the chain with different impact and some examples for R are given in table 8 below (analysis based on hexadecanoic acid).

Table 8:

| R= | D (log(D)) | R= | D(log(D)) |
|---|---|---|---|
| Vinyl | +0.4 | Methoxymethyl- | -1 |
| chloride | -0.2....-0.5 | Ethoxymethyl- | -0.5 |
| Fluoride | -0.9 | Ethoxyethyl- | 0 |
| Bromide | -0.35.... +0.2 | Keto- | -2.2 .... -1.7 |
| Hydroxyl | -1.2 ... 2.2 | | |

The substituents itself are not pH-responsive and therefore do not contribute to the pH-response of the TEE. Also, the impact of R is independent of the pH. However, as pointed out before R may influence the pka of the hydrophilic element, thereby changing the amplitude of log(D) between physiological pH and pH4...5 .

In still other aspects, heteroatoms may be part of the main chain of the hydrophobic element. In other aspects, the main chain may comprise non-saturated bonds. In still other aspects, the main chain may comprise heteroatoms in combination with subsitutions in the side chain. Such changes may influence the logD of the TEE and some variations

for the main chain have been analyzed for hexadecanoic acid (table 9).

Table 9:

| double bonds (-2H) | | | | ether in main chain (PEG) | | | | ether in main chain (PPG) | | | |
|---|---|---|---|---|---|---|---|---|---|---|---|
| # subs | pH 4,0 | pH7,4 | ☐ | #subs | pH 4,0 | pH7,4 | ☐ | #subs | pH 4,0 | pH7,4 | ☐ |
| 0 | 7,09 | 4,56 | -2,53 | 0 | 7,09 | 4,56 | -2,53 | 0 | 7,09 | 4,56 | -2,53 |
| 1 | 7,11 | 4,59 | -2,52 | 1 | 5,16 | 2,54 | -2,62 | 1 | 5,51 | 2,88 | -2,63 |
| 2 | 6,58 | 3,99 | -2,59 | 2 | 3,21 | 0,57 | -2,64 | 2 | 3,9 | 1,26 | -2,64 |
| 3 | 5,95 | 3,09 | -2,86 | 3 | -0,69 | -3,33 | -2,64 | 3 | 2,3 | -0,34 | -2,64 |

A double bond therefore reduces logD by about -0.4 to 0.5 units, ether bonds in the main chain as found in repetitive strucutres derived from poly-ethylenglycol reduce logD by about 2.5 units, said effect being reduced to about 1.6 units by addition of an additional methyl group next to the ether bond, such as in the repetitive structures from polypropylenglycol.

Other substitutions on the main chain may further change the logD of the chain with different impact and some examples are given in table 10 below (analysis based on hexadecanoic acid).

Table 10:

| element | D (log(D)) | element | D(log(D)) |
|---|---|---|---|
| -CH2- | Reference | -S- | -1,5 |
| -NH- (Aza) | -6 .... -9 | -S-S- | -1.1 |
| -NH-CO- (Amide) | -7.5 ... -11 | | |
| -O-CO- (Ester) | -2,7 | | |

The substituents itself are not pH-responsive and therefore do not contribute to the pH-response of the TEE. Also, the impact of R is independent of the pH. However, as pointed out before R may influence the pka of the hydrophilic element, thereby changing the amplitude of log(D) between physiological pH and pH9...5 .

The examples and analysis presented above give guidance for practicing the invention, in particular to identify structural elements as TEE's. Isoforms and position isomers are within the disclosure of the current invention. As mentioned earlier in this disclosure, neighbouring effects between substituents may occur. However, these effects are known to the skilled artisan and are described in the standard chemical literature,in databases or are part of chamical software such as ACD/Labs and others.

Ring systems

In some aspects, the hydrophobic element may form a cyclic structure such as cycloalkanes, cycloalkenes or cycloalkines or aromatic ring structures. A few representations of cyclic elements have been analyzed in the table below and more cyclic elements can be developed from known structural contributions of other elements. It is of course possible to further substitute the cyclic elements, in particular with the groups analyzed above.

Table 11:

| | Cyclic backbone | | | |
|---|---|---|---|---|
| | cpd | pH 4,0 | pH7,4 | Δ |
| stearic acid | | 8,15 | 5,62 | -2,53 |
| 4-undecyl cyclohexanoic acid | | 7,54 | 5,22 | -2,32 |
| 4-undecyl cyclohexenoic acid | | 7,12 | 4,39 | -2,73 |
| p-undecyl benzoic acid | | 7,51 | 4,87 | -2,64 |

Sterols

In a specific variant of the invention, the ring systems of the hydrophobic elements may be more complex ring

systems such as in sterols. Again, further substitutions may be present at the sterol backbone and the analysis shown below is detailing some aspects of logD for natural occuring derivatives of sterols, e.g. hydroxyl group substitutions at positions 3,7 and 12 of the sterane backbone.
Also, the orientation of the sterol in the TEE may be different and a presence of the carboxyl group at position 26 such as in bile acids or grafted onto position 3 such as in cholesterolhemisuccinate (CHEMS) represent some instant examples (tables 12 and 13).

Table 12: Analysis of logD for bile acid derivatives used as TEE's. For analytical purposes, the 3' hydroxyl group was assumed to be methylated to model a potential drug linkage

| **bile acids** | **OH in backbone** | | | |
|---|---|---|---|---|
| (3'cholesterols) | **#subs** | **pH 4,0** | **pH7,4** | Δ |
| 3' methoxy cholate | 3 | 3,18 | 0,64 | -2,54 |
| 3' methoxy deoxycholate | 2 | 5,23 | 2,69 | -2,54 |
| 3' methoxy dideoxycholate | 1 | 7,27 | 4,73 | -2,54 |

Table 13: Analysis of logD for CHEMS derivatives used for TEE's. For analytical purposes, the carboxyl group in position 26 was esterified with methanol to model a potential drug linkage. In this analysis, the 3' position is esterified with succinic acid, thereby providing an unsubstituted carboxyl group as the hydrophilic element of the TEE.

| **CHEMS derivatives** | **OH in backbone** | | | |
|---|---|---|---|---|
| (26' cholesterols) | **# subs** | **pH 4,0** | **pH7,4** | Δ |
| 26' methyl cholate | 3 | 3,65 | 1,01 | -2,64 |
| 26' methyl deoxycholate | 2 | 5,69 | 3,06 | -2,63 |
| 26' methyl dideoxycholate | 1 | 7,73 | 5,1 | -2,63 |

B. TEE's based on barbituric acids and xanthins

The considerations above can of course be transferred to TEE's comprising structurally different hydrophilic elements. Contributions of structural elements in the hydrophobic section of the TEE are close if not identical irrespective of the chemical nature of the hydrophilic element or elements. Some specific chemical representations of TEE's comprising barbituric acid or xanthine may illustrate the construction of such TEE's without limiting this part of the disclosure.

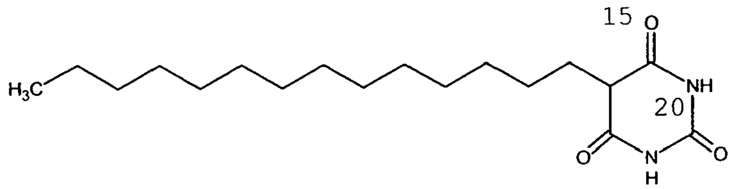

(17)

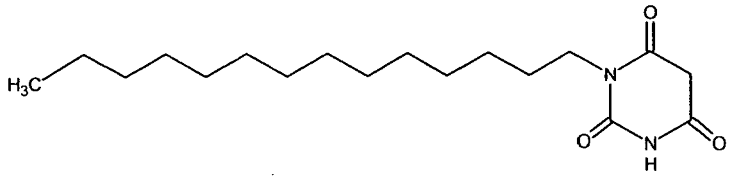

(18)

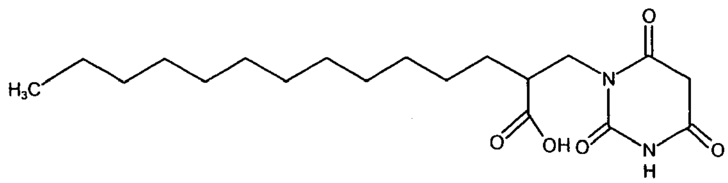

(19)

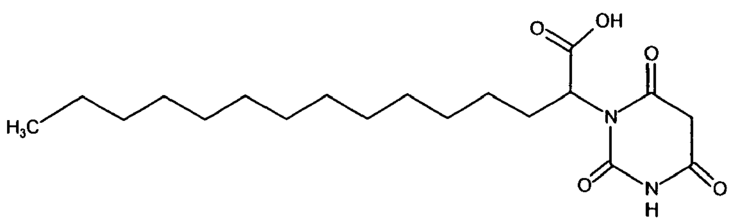

(20)

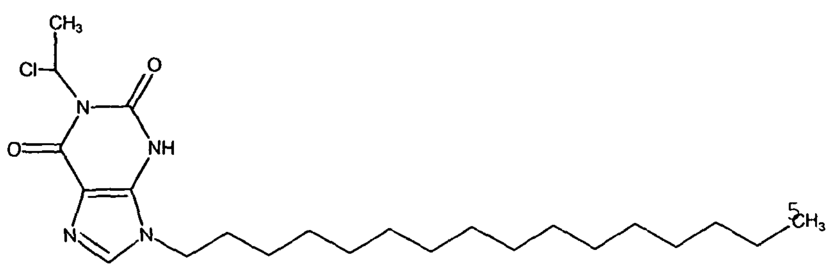

(21)

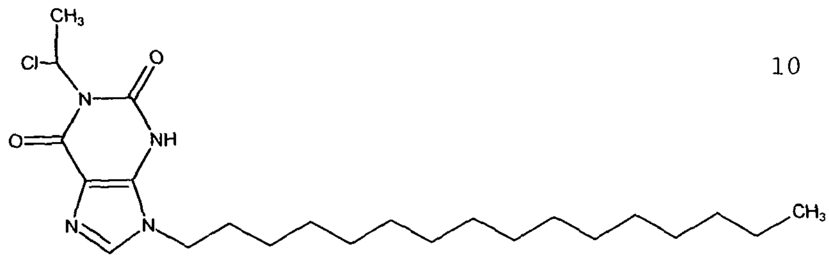

10 (22)

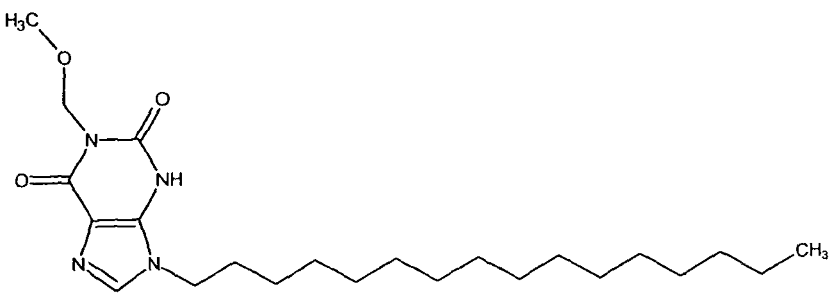

(23)

The position of the hydrophilic element within the TEE structure may vary. In some aspects, the hydrophilic element is located distal from the link between molecule and TEE. In other aspects, the hydrophilic element is located central within the TEE.

Use of TEE's

The TEE's can directly be linked to active pharmaceutical ingredients such as small molecules, but also to proteins, peptides, carbohydrates or nucleic acids. The conjugation with the substance to be transported can be achieved by means of chemical bonds, physical attraction or by other interactions.

A conjugation via chemical bonds can be achieved by any chemical reaction, which may lead to linker groups including, without limitation, -O-, -S-, - NH-, -NR-, -N(H)C(O)-, -C(O)O-, -OC(O)N(H)-, -C(O)-, -C(O)-N(H)-, - N(H)-C (O)-O-, -CH=N-, -O-C(O)-, -N=CH-, -OP(O)(OH)O-, -P (O) (OH) O-, - OP(O)(OH)-, -OS(O)(O)O-, -S(O)(O)O-, -OS(O) (O)- and/or -S-S-.

Methods of synthesize such conjugates comprising a transfection enhancer element as described above include coupling reactions that are well-known to those skilled in the art and may vary depending on the starting material and coupling component employed. Typical reactions are esterification, amidation, etherification, or reductive amination.

TEE's can also be conjugated to carrier systems sequestering the actual active ingredient. Such carrier systems may comprise polymers or copolymers or block-polymers of various chemistries. Such carriers may further comprise supramolecular aggregates of polymers, e.g. polymer based beads. In one aspect of the invention such carriers can be lipid aggregates or liposomes.

Other carrier systems may comprise biocompatible carriers suitable for the transport of small molecules, peptides, proteins or nucleic acids. Examples for such carriers include, without limitation cationic polymer based systems such as polyethylenimins of linear or branched type (e.g. Boussif et al, Proc. Natl. Acad. Sci., 92(16), 7297-7301, 1995 or Godbey et al., J. Control. Release, 60(2-3), 149-160, 1999), chitosan and derivatives thereof (e.g. Janes et al., Adv. Drug Deliv. Rev., 47(1), 83-97, 2001), cationic cyclodextrins and derivatives thereof, some of them being disclosed in Hu-Lieskovan et al., Cancer Res., 65(19), 8984-8992, 2005. Examples may further include carriers based on gelatin, collagen or atelocollagen, some of them being disclosed e.g. in Kaul et al., Pharm. Res., 22(6), 951-961, 2005) or Sano et al., Adv. Drug Deliv. Rev., 55(12), 1651-1677, 2003. It is possible to use carriers derived from viral capsids for the delivery of polynucleotides or oligonucleotides and TEE's of this invention can be combined with viral capsids, such combination can be done before or after assembly of the capsid.

In one aspect of the invention, one or more TEE's can be grafted on the carrier systems or polymers using chemical conjugation techniques and methods for carrying out such couplings have extensively been reported in the literature, e.g. by G. T. Hermanson, Bioconjugate Techniques, Academic Press, 1996.

In another aspect of the invention, one or more TEE's may be complexed with the carrier systems using molecular recognition, e.g. between biotin and biotin binding proteins, between two complementary or partially complementary oligonucleotides or between cyclodextrins and molecules fitting the binding pockets of the cyclodextrin such as various lipids or detergents as disclosed in DeGrip et al., Biochem. J., 330, 667-674, 1998, sterols or adamantane units as disclosed in WO 06/105361. In another aspect, one or more TEE's may be complexed with carrier systems using ionic or electrostatic interaction.

In yet another aspect, one or more TEE's may become an integral part of the carrier system. In one embodiment, TEE's further comprising a polycationic element are combined with polyanions to form a precipitate, a complex controlled in size or a stoichiometric associate. Examples for polycationic elements include without limitation polyamines, eg. spermine, thermine, spermidine, putrescine, polylysine or polyarginine. It is known in the art that polyamines form complexes with oligonucleotides or polynucleotides. It is also known that derivatives of spermine, spermidine or putrescine, such as for example lipopolyamines still are able to form complexes with oligonucleotides or polynucleotides. Lipopolyamines include, without limitation, cholesteryl polyamine carbamates or the commercially available lipopolyamine compounds DOSPER, DOGS or DOSPA. TEE derivatives for such use include, but are not limited to the compounds 24 - 28 (table 14).

Table 14: Compounds 24-28

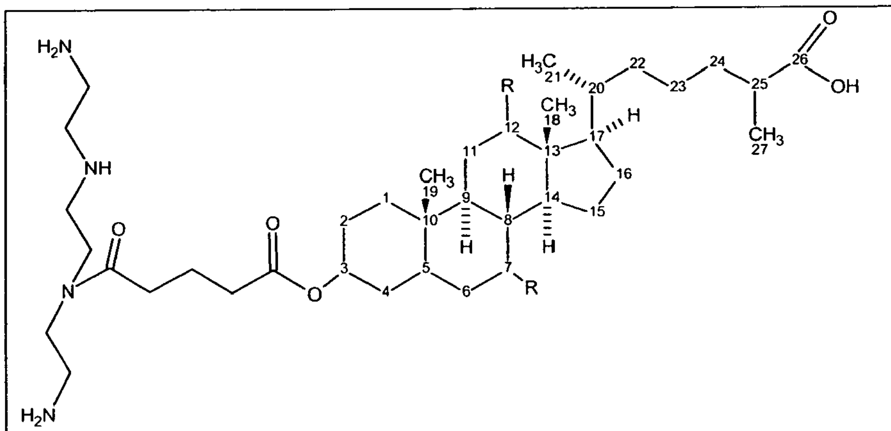

Compound (24), R is -H or -OH

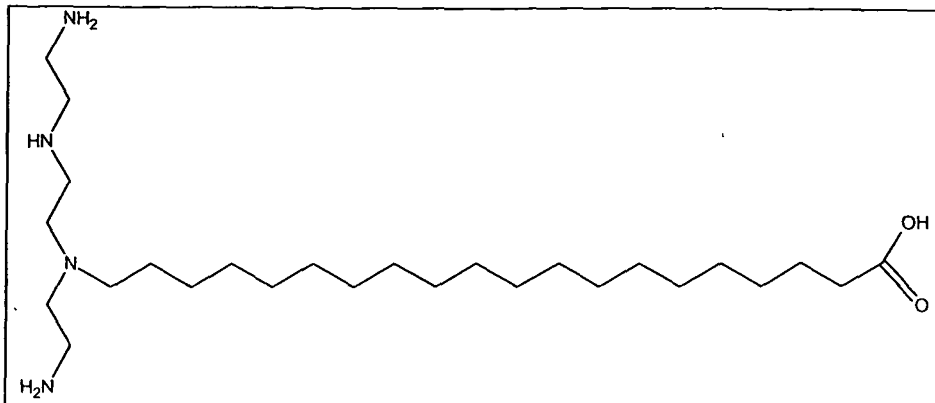

Compound (25)

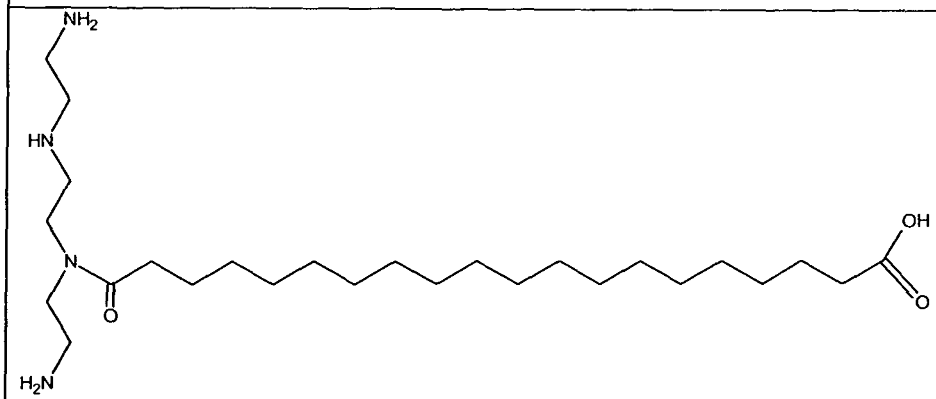

Compound (26)

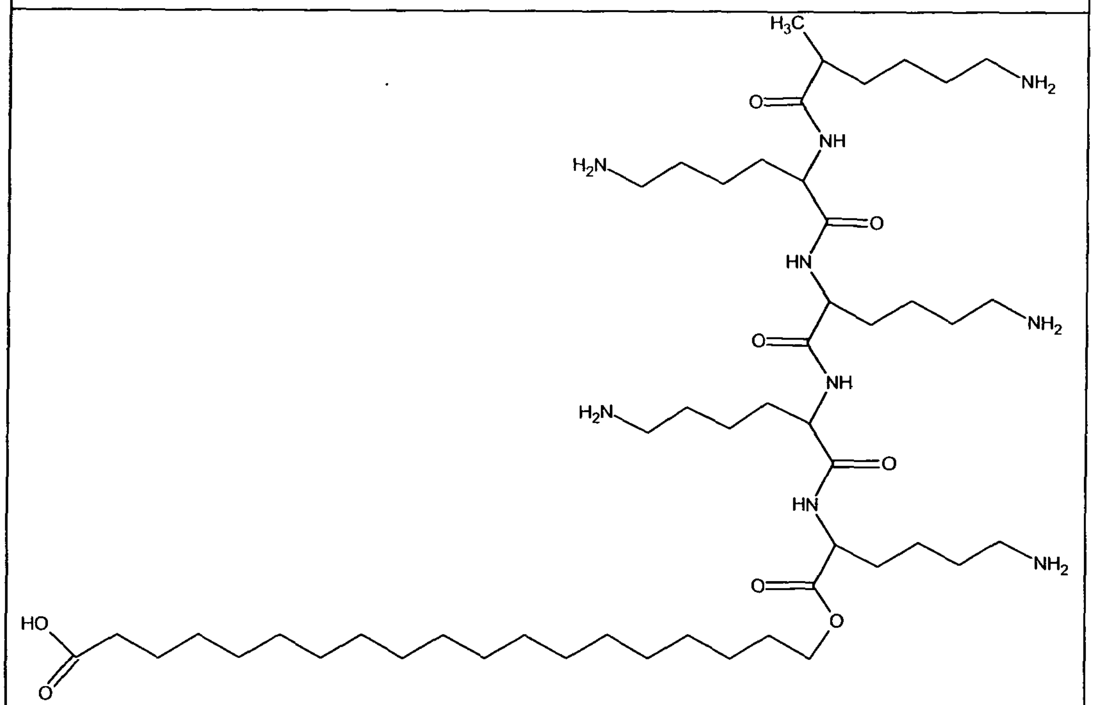

Compound (27)

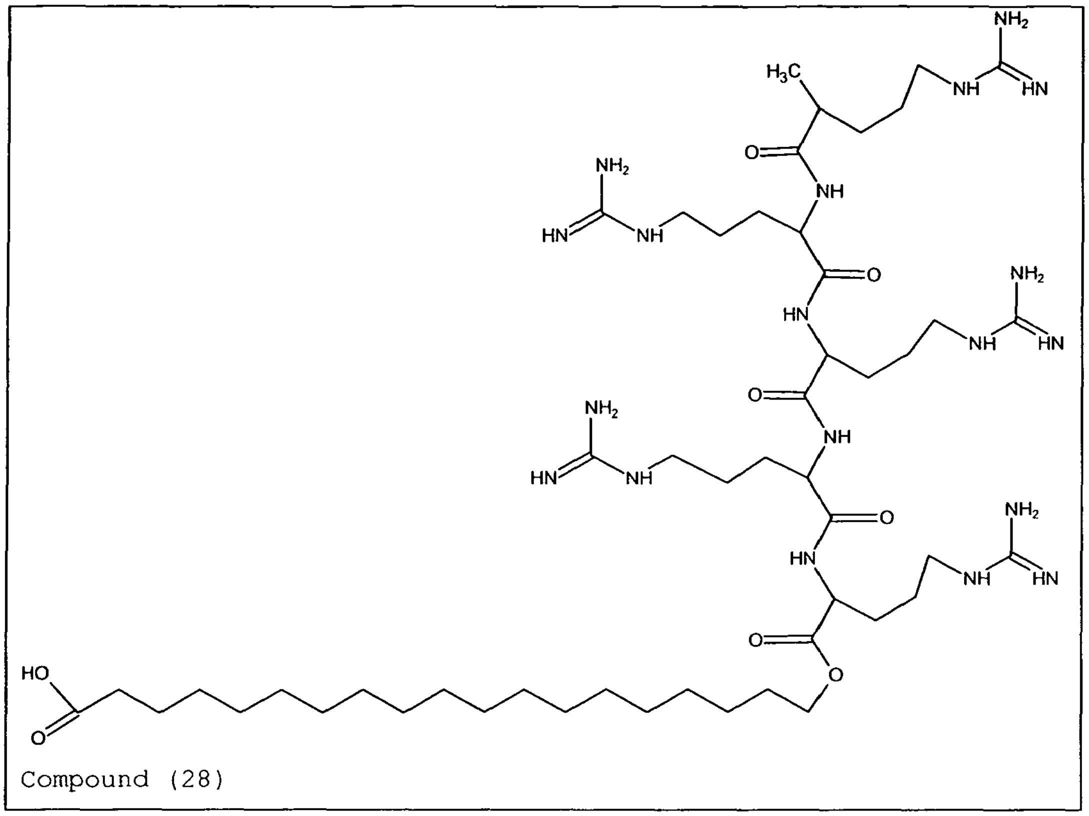

Compound (28)

In another aspect of the invention the active ingredient to be transported can be a polymer or oligomer itself, e.g. a nucleic acid, a peptide or a protein.

Nucleic Acids modified with TEE's

In one embodiment of the invention, TEE's are grafted onto nucleic acids. In one aspect of such embodiment, TEE's are grafted onto the phosphate backbone of nucleic acids. In one variant, TEE's for such use comprise straight alkyl chains of 6 to 16 C-atoms and further comprise a single carboxyl group. In one specific embodiment, the carboxyl group is situated at the terminal end of the alkyl chain, as in compound (29).

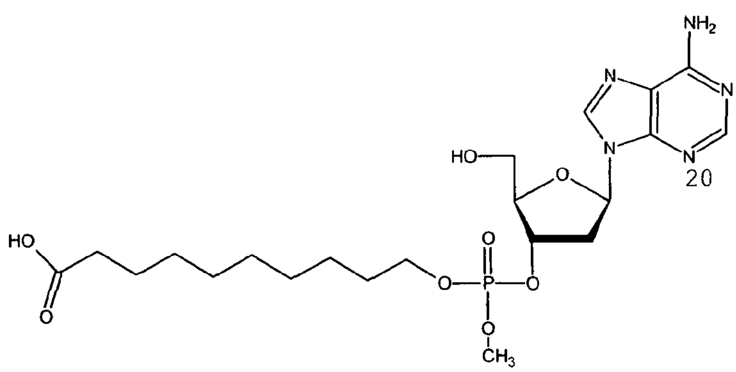

Compound (29):
C10-carboxyl-deoxyadenin.
The TEE is decanoic acid.

Of course, any position at the nucleobases or derivatives thereof is a potential graft position of a TEE, including the 2'O- or 2'S-positions of the nucleosides, the heterocyclic base of the nucleosides or the internucleoside linkages, e.g. the phosphate backbone. In one embodiment of the invention TEE's are grafted onto nucleic acids in such a way that base pairing and consequently hybridization with target sequences is not interfered. In a preferred embodiment TEE's are

grafted onto the phosphate backbone of nucleic acids.

In some embodiments of the invention the TEE moieties may be attached to the nucleobases via linking groups. Nucleobases in combination with long chain carboxylic acids are known in the state of the art, but were used for conjugating nucleic acids with other substances, e.g. for targeting cellular receptor. Consequently, only a limited number of such TEE modified nucleobases were incorporated into oligonucleotides or polynucleotides. For example oligonucleotides having pre-activated carbonyl linkers are disclosed in US 6320041. Oligonucleotides having 5'carboxyl linker of 10 C atoms are commercially available at TriLink Biotechnologies, San Diego, USA.

In other aspects of the invention, TEE's of different structural origin can be used to enhance the transfection of nucleic acids.

In other aspects of this disclosure, nucleobases are absent and TEE's are grafted directly onto a phosphoribose or phosphodexyribose backbone, replacing the former nucleobases at C1. Examples for such structures include, without limitation the following chemical representations (Compound (30):

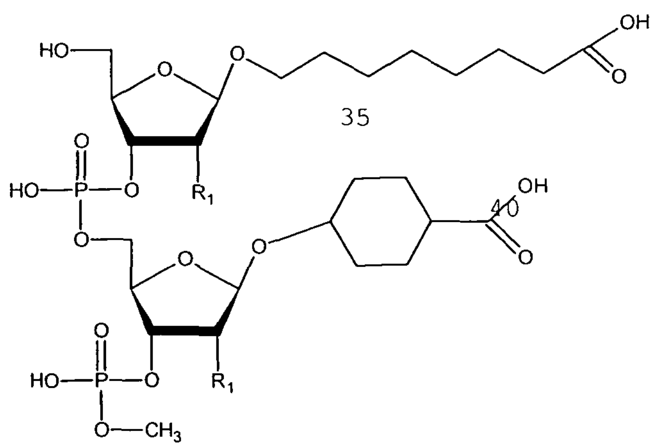

Compound (30)

This invention teaches the use of one or multiple TEE's grafted onto a single oligonucleotide or polynucleotide for enhancing the transfection of such substances. This invention also discloses conjugates of oligonucleotides or polynucleotides with one or more TEE's selected from the different chemical representations given throughout this disclosure. In some aspects of the invention conjugates of oligonucleotides with one TEE may comprise a TEE other than a saturated, straight chain 1-carboxylic acid. In other aspects of the invention the use of transfection enhancer elements comprising saturated, straight chain 1-carboxylic acid for the in vivo, in vitro or ex vivo transfection of oligonucleotides may be preferred.

In preferred embodiments, 2 or more TEE's with the chemical representations given throughout the invention are conjugated to the oligonucleotide. In some aspects of such embodiments, the TEE's are alkylcarboxylic acids with 6 or more carbon atoms, in more preferred embodiments of such aspect, the TEE's comprise 8 to 16 carbon atoms. In other aspects of such embodiments the TEE's may be sterol-based, including but not limited to bile acid derivatives as shown below (Compounds (31) and (32)).

In more preferred embodiments, 4 or more TEE's with the chemical representations given throughout the invention are conjugated to the oligonucleotide. In some aspect of such embodiments, the TEE's are alkylcarboxylic acids with 6 or more carbon atoms, in more preferred embodiments of such aspect, the TEE's comprise 8 to 16 carbon atoms. In other aspects of such embodiments the TEE's may be sterol-based, including but not limited to bile acid derivatives as shown below (Compounds (31) and (32)).

In other preferred embodiments, 6 or more TEE's with the chemical representations given throughout the invention are conjugated to the oligonucleotide. In some aspect of such embodiments, the TEE's are alkylcarboxylic acids with 6 or more carbon atoms, in more preferred embodiments of such aspect, the TEE's comprise 8 to 16 carbon atoms. In other aspects of such embodiments the TEE's may be sterol-based, including but not limited to bile acid derivatives as shown below (Compounds (31) and (32)).

In some embodiments, all nucleobases in the oligonucleotide are modified with TEE's with the chemical representations given throughout the invention. In some aspect of such embodiments, the TEE's are alkylcarboxylic acids with 6 or more carbon atoms, in more preferred embodiments of such aspect, the TEE's comprise 8 to 16 carbon atoms. In other aspects of such embodiments the TEE's may be sterol-based, including but not limited to bile acid derivatives as shown below (Compounds (31) and (32)).

In some preferred embodiments, no more than 2/3 of all nucleobases in the oligonucleotide are modified with TEE's

with the chemical representations given throughout the invention. In some aspect of such embodiments, the TEE's are alkylcarboxylic acids with 6 or more carbon atoms, in more preferred embodiments of such aspect, the TEE's comprise 8 to 16 carbon atoms. In other aspects of such embodiments the TEE's may be sterol-based, including but not limited to bile acid derivatives as shown below (Compounds (31) and (32)).

In some other preferred embodiments, no more than 1/3 of all nucleobases in the oligonucleotide are modified with TEE's with the chemical representations given throughout the invention. In some aspect of such embodiments, the TEE's are alkylcarboxylic acids with 6 or more carbon atoms, in more preferred embodiments of such aspect, the TEE's comprise 8 to 16 carbon atoms. In other aspects of such embodiments the TEE's may be sterol-based, including but not limited to bile acid derivatives as shown below (Compounds (31) and (32)).

In yet other embodiments of the invention only nucleobases at the flanks of the oligonucleotides or polynucleotides are modified with TEE's leading to gapmer structures. In a preferred aspect of this embodiment no more than 2/3 of all nucleobases were modified with TEE's with the chemical representations given throughout the invention. In some aspect of such embodiments, the TEE's are alkylcarboxylic acids with 6 or more carbon atoms, in more preferred embodiments of such aspect, the TEE's comprise 8 to 16 carbon atoms. In other aspects of such embodiments the TEE's may be sterol-based, including but not limited to bile acid derivatives as shown below (Compounds (31) and (32)).

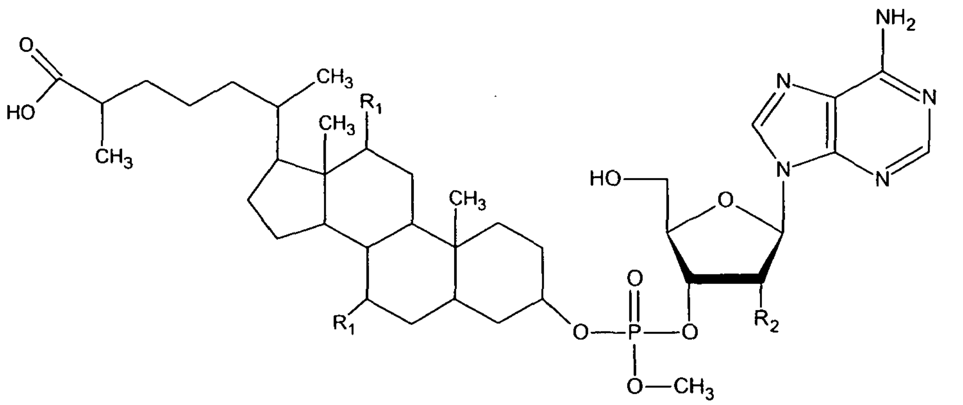

Compound (31): Nucleotides modified with a bile acid or derivative thereof. R1 and R2 may independently be H, OH, methyl-, ethyl-, propyl-, isopropyl-, methoxy-, ethoxy-, methoxymethyl-, ethoxymethyl-, methoxyethyl-, ethoxyethyl- and vinyl- or halogen. In further preferred variants, R1 is -H or -OH or mixtures of both and R2 is -H, -OH, -OMe or methoxyethyl.

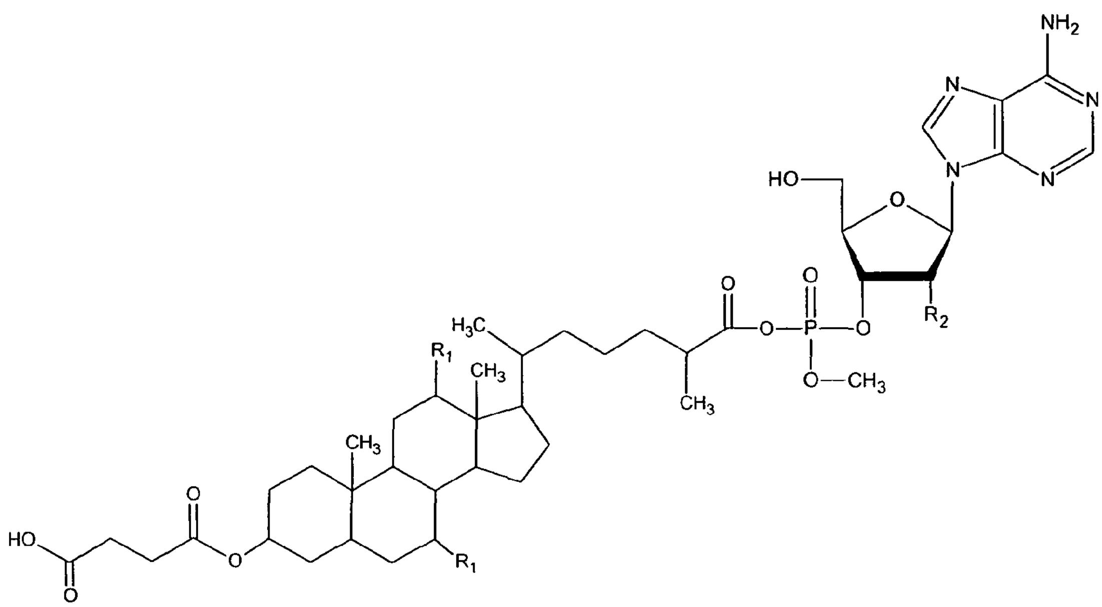

Compound (32): Nucleotides modified with a bile acid or derivative thereof. R1 and R2 may independently be H, OH,

methyl-, ethyl-, propyl-, isopropyl-, methoxy-, ethoxy-, methoxymethyl-, ethoxymethyl-, methoxyethyl-, ethoxyethyl- and vinyl- or halogen. In further preferred variants, R1 is -H or -OH or mixtures of both and R2 is -H, -OH, -OMe or methoxyethyl.

All nucleobases can be used in conjugation with the TEE's and the physicochemical differences between the individual nucleobases are rather small compared to the impact of the modifying TEE's. The logD values for structural elements of an oligonucleotide can be predicted using known values from the literature and standard algorithms. The carboxylmethyl derivative of a nucleobase has comparable values of logD(7.4) to the unmodified phosphate variant (~ -5) and every methylene group added to the hydrophobic chain of the TEE raises the logD about 0.5 units. Figure 5 gives a more detailed analysis for the different nucleobases. It becomes apparent from the figure that the carboxyl groups from the TEE increase the logD with about 2 to 2.5 units when exposed to the slightly acidic pH.

Figure 6 gives a more detailed analysis for a short oligonucleotide backbone fragment being fully modified with C8 carboxyl TEE's. The structures for the analysis are derived from compound 33.

Compound (33):

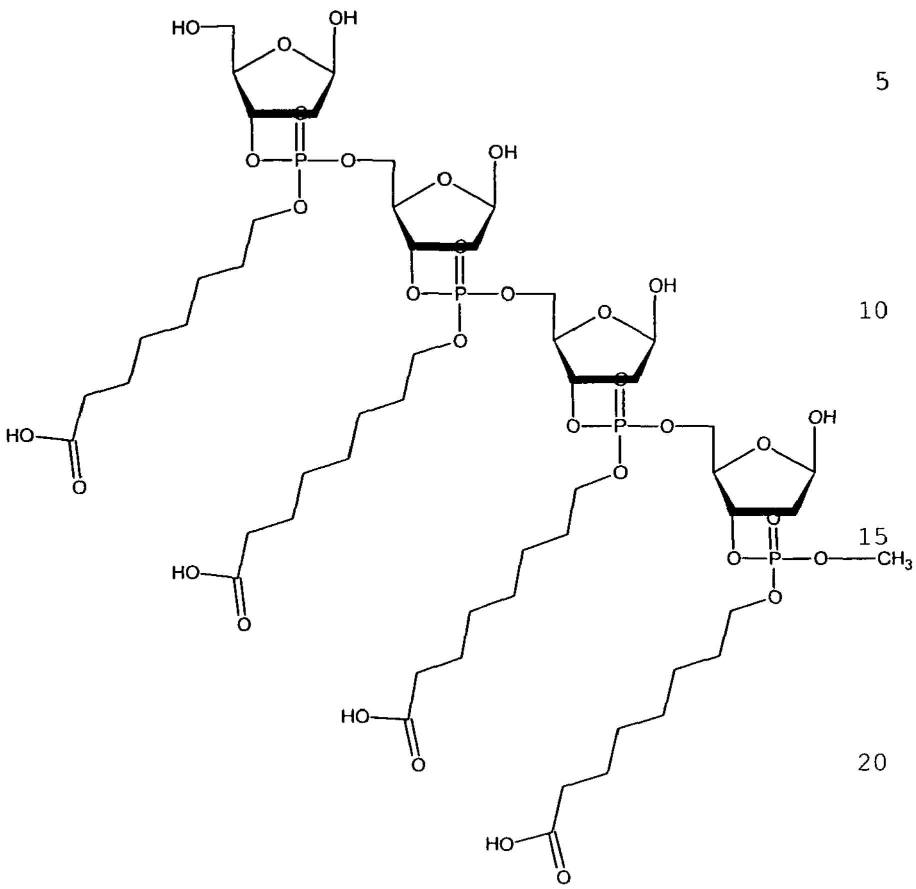

In contrast to the unmodified structures, the TEE modified backbone is already less hydrophobic at neutral pH, but still very hydrophilic. More importantly and in contrast to the unmodified form, the TEE modified structure is now sensitive to a shift in pH between 4 and 7. Under slightly acidic conditions the structure has very limited hydrophilicity and is now able to diffuse through biological membrane. Moreover, any extension of the fragment towards longer chains, although increasing the hydrophilicity at pH7, does increase the hydrophobicity at pH4 to a very limited extent.

Use of TEE's for the improvement of transfection is conceptually different from a mere hydrophobic modification of the nucleic acids. Hydrophobic modification has been disclosed e.g. by Letsinger et al. in US 4958013 or Proc. Natl. Acad. Sci., 86, 6553-6556, 1989 or by Manoharan et al. in US 6,153,737 and US 6,753,423 in combination with single stranded oligonucleotides and was also used to deliver siRNA in vivo (Soutschek et al., Nature, 432(7014), 173-178, 2004). A mere hydrophobic modification does not respond to changes in pH of the environment and is therefore different from the TEE's of the invention.

Practicing the invention and use of TEE's is not limited to otherwise unmodified oligonucleotides and TEE's can be grafted on many different nucleotides, oligonucleotides or nucleic acids or polynucleic acids.

Without being limited to such use, the TEE's described in the present invention are well suited for modify nucleic acid-based drugs such for example as oligonucleotides, polynucleotides or DNA plasmids. These drugs may be classified into nucleic acids that encode one or more specific sequences for proteins, polypeptides or RNAs and into oligonucleotides that can specifically regulate protein expression levels or affect the protein structure through interference with splicing, artificial truncation and others.

In some embodiments of the present invention, therefore, the nucleic acid-based therapeutic may comprise a nucleic acid that is capable of being transcribed in a vertebrate cell into one or more RNAs, which RNAs may be mRNAs, shRNAs, miRNAs or ribozymes, wherein such mRNAs code for one or more proteins or polypeptides. Such nucleic acid therapeutics may be circular DNA plasmids, linear DNA constructs, like MIDGE vectors (Minimalistic Immunogenically Defined Gene Expression) as disclosed in WO 98/21322 or DE 19753182, or mRNAs ready for translation (e.g., EP 1392341).

In another embodiment of the invention, oligonucleotides may be used that can target existing intracellular nucleic acids or proteins. Said nucleic acids may code for a specific gene, such that said oligonucleotide is adapted to attenuate or modulate transcription, modify the processing of the transcript or otherwise interfere with the expression of the protein. The term "target nucleic acid" encompasses DNA encoding a specific gene, as well as all RNAs derived from such DNA, being pre-mRNA or mRNA. A specific hybridisation between the target nucleic acid and one or more oligonucleotides directed against such sequences may result in an inhibition or modulation of protein expression. To achieve such specific targeting, the oligonucleotide should suitably comprise a continuous stretch of nucleotides that is substantially complementary to the sequence of the target nucleic acid.

Oligonucleotides fulfilling the abovementioned criteria may be built with a number of different chemistries and topologies. The oligonucleotides may comprise naturally occurring or modified nucleosides comprising but not limited to DNA, RNA, locked nucleic acids (LNA's), 2'0-methyl RNA (2'Ome), 2' O-methoxyethyl RNA (2'MOE) in their phosphate or phosphothioate forms or Morpholinos or peptide nucleic acids (PNA's).
Oligonucleotides may be single stranded or double stranded.

The mechanisms of action of oligonucleotides may vary and might comprise effects on splicing, transcription, nuclear-cytoplasmic transport and translation, amongst others.

In a preferred embodiment of the invention single stranded oligonucleotides may be used including but are not limited to DNA-based oligonucleotides, locked nucleic acids, 2'-modified oligonucleotides and others, commonly known as antisense oligonucleotides. Backbone or base or sugar modifications may include but are not limited to Phosphothioate DNA (PTO), 2'O-methyl RNA (2'Ome), 2' O- methoxyethyl-RNA (2'MOE), peptide nucleic acids (PNA), N3'-P5' phosphoamidates (NP), 2'fluoroarabino nucleic acids (FANA), locked nucleic acids (LNA), Morpholine phosphoamidate (Morpholino), Cyclohexene nucleic acid (CeNA), tricyclo-DNA (tcDNA) and others. Moreover, mixed chemistries are known in the art, being constructed from more than a single nucleotide species as copolymers, block-copolymers or gapmers or in other arrangements.

In addition to the aforementioned oligonucleotides, protein expression can also be inhibited using double stranded RNA molecules containing the complementary sequence motifs. Such RNA molecules are known as siRNA molecules in the art (e.g., WO 99/32619 or WO 02/055693). Again, various chemistries were adapted to this class of oligonucleotides. Also, DNA / RNA hybrid systems are known in the art.

In another embodiment of the present invention, decoy oligonucleotides can be used. These double stranded DNA molecules and chemical modifications thereof do not target nucleic acids but transcription factors. This means that decoy oligonucleotides bind sequence-specific DNA-binding proteins and interfere with the transcription (e.g. Cho-Chung, et al. in Curr. Opin. Mol. Ther., 1999).

In a further embodiment of the invention oligonucleotides that may influence transcription by hybridizing under physiological conditions to the promoter region of a gene may be used. Again various chemistries may adapt to this class of oligonucleotides.

In a further alternative of the invention, DNAzymes may be used. DNAzymes are single-stranded oligonucleotides and chemical modifications therof with enzymatic activity. Typical DNAzymes, known as the "10-23" model, are capable of cleaving single-stranded RNA at specific sites under physiological conditions. The 10-23 model of DNAzymes has a catalytic domain of 15 highly conserved deoxyribonucleotides, flanked by 2 substrate-recognition domains complementary to a target sequence on the RNA. Cleavage of the target mRNAs may result in their destruction and the DNAzymes recycle and cleave multiple substrates.

In another embodiment of the invention, ribozymes can be used. Ribozymes are single-stranded oligoribonucleotides and chemical modifications thereof with enzymatic activity. They can be operationally divided into two components, a conserved stem-loop structure forming the catalytic core and flanking sequences which are reverse complementary to sequences surrounding the target site in a given RNA transcript. Flanking sequences may confer specificity and may generally constitute 14-16 nt in total, extending on both sides of the target site selected.

In a further embodiment of the invention aptamers may be used to target proteins. Aptamers are macromolecules composed of nucleic acids, such as RNA or DNA, and chemical modifications thereof that bind tightly to a specific molecular target and are typically 15-60 nt long. The chain of nucleotides may form intramolecular interactions that fold the molecule into a complex three-dimensional shape. The shape of the aptamer allows it to bind tightly against the surface of its target molecule including but not limited to acidic proteins, basic proteins, membrane proteins, transcription factors and enzymes. Binding of aptamer molecules may influence the function of a target molecule.

All of the above-mentioned oligonucleotides may vary in length between as little as 5, preferably between 8 and 50 nucleotides. The fit between the oligonucleotide and the target sequence is preferably perfect with each base of the oligonucleotide forming a base pair with its complementary base on the target nucleic acid over a continuous stretch of the abovementioned number of oligonucleotides. The pair of sequences may contain one or more mismatches within the said continuous stretch of base pairs, although this is less preferred. In general the type and chemical composition of such nucleic acids is of little impact for the performance of the invention and the skilled artisan may find other types of oligonucleotides or nucleic acids suitable for use with this invention.

A further aspect of the invention relates to pharmaceutical compositions comprising conjugates of nucleic acids with pH-responsive transfection enhancer elements (TEE's). The pharmaceutical composition of the present invention may be formulated in a suitable pharmacologically acceptable vehicle. Vehicles such as water, saline, phosphate buffered saline and the like are well known to those skilled in the art for this purpose.

In some embodiments said pharmaceutical compositions may be used for the treatment or prophylaxis of inflammatory, immune or autoimmune disorders and/or cancer of humans or non-human animals.

A yet further aspect of the present invention relates to methods for the treatment of human or non-human animals in which said pharmaceutical composition comprising conjugates of nucleic acids with transfection enhancer elements is targeted to a specific organ or organs, tumours or sites of infection or inflammation.

In the drawings:

Figure 1 shows the logD response of compound 11-14

Figure 2 shows the relation between logD and pH for different hydrophiles in TEE structures. All hydrophilic elements are located at the terminal position of the alkyl chain. LogD starts to decrease at pH values roughly equal to pKa, such decrease being limited by ion pair formation at physiological ionic strenght.

Figure 3 shows standardized curves for logD wherein pH-pKa is used as x-axis for different hydrophiles in TEE structures. All hydrophilic elements are located at the terminal position of the alkyl chain. 3-cholor octanoic acid is identical with octanoic acid and not shown in the plot.

Figure 4 shows an analysis of the increment of logD vs. pH. The curve was standardized using pH-pKa as a common descriptor that is independent from the pKa of the individual hydrophilic elements.

Figure 5 shows the impact of alkyl chain length in the TEE for different nucleobases and phosphodeoxyribose (mononucleotide form, phosphate terminated with methyl as in compound 22). All species analyzed respond to acidification of the medium with an increase of the logD values of about 2.3 units. Nucleobases modified with TEE's longer than 8 carbon atoms become hydrophobic at pH 4.5
Such shift dramatically ameliorates the hydrophobicity of the oligonucleotide and facilitates transfection of the entire structure.

Figure 6 shows the impact of the addition of TEE's to oligophosphoribose of different length. (A) Oligophosphoriboses having 1, 2, 3 or 4 repetitive units were analyzed towards their logD profile. Each phosphoribose unit contributes a log D of about -3.3units, making it impossible for longer oligonucleotides to cross biological membranes. Also, compounds with a phosphate backbone not modified with a TEE do not response to changes in the pH between pH 3 and pH 10.
(B) Oligophosphoriboses having 1, 2, 3 or 4 repetitive units and modified with TEE's comprising octylcarboxylic groups were analyzed towards their logD profile. The resulting conjugates are less hydrophilic at neutral pH and each unit now contributes only 2.2 units of logD. More importantly, the TEE modified oligophosphoribose responds strongly towards an acidification of the medium and a compound with very little hydrophilic character results. Also, chain extension do no longer contribute to the hydrophilic character, as the impact on logD at pH 4.5 is only 0.25 units per extra unit of the oligomer.

## Claims

1. Conjugates of nucleic acids with two or more pH-responsive transfection enhancer elements (TEE's) with the general structure (I)

Hydrophobic element - pH-responsive hydrophilic elements (I)

2. Conjugates of nucleic acids with two or more pH-responsive transfection enhancer elements (TEE's) as claimed in claim 1 wherein said pH-responsive hydrophilic element is located distal from the link between said nucleic acid and TEE.

3. Conjugates of nucleic acids with two or more pH-responsive transfection enhancer elements (TEE's) as claimed in claim 1 wherein said pH-responsive hydrophilic element is located central within the TEE.

4. Conjugates of nucleic acids with two or more pH-responsive transfection enhancer elements (TEE's) as claimed in any of claim 1 to 3 wherein said pH-responsive hydrophilic element comprises weak acids having a pka of between 2 and 6, preferred of between 3 and 5.

5. Conjugates of nucleic acids with two or more pH-responsive transfection enhancer elements (TEE's) as claimed in claim 4 wherein said weak acids are selected from the group comprising carboxyl groups, barbituric acid and derivatives thereof, xanthine and derivatives thereof, pyrimidines and derivatives thereof, uracils and derivatives thereof.

6. Conjugates of nucleic acids with two or more pH-responsive transfection enhancer elements (TEE's) as claimed in any of claim 1 to 3 wherein said pH-responsive hydrophilic element is a zwitterionic structure comprising a combination of weak or strong acidic groups with weak bases having a pka of between 3 and 8, preferred of between 4.5 and 7.

7. Conjugates of nucleic acids with two or more pH-responsive transfection enhancer elements (TEE's) as claimed in claim 6 wherein said zwitterionic structure may be formed from an anionic group and a heterocyclic nitrogen atom as cationic group.

8. Conjugates of nucleic acids with two or more pH-responsive transfection enhancer elements (TEE's) as claimed in any of claims 1 to 7 wherein said pH-responsive hydrophilic element may further be substituted with structural elements selected from the group comprising hydroxymethyl-, hydroxyethyl-, methoxymethyl-, methoxyethyl-, ethoxymethyl-, ethoxyethyl-, thiomethyl-, thioethyl-, methylthiomethyl-, methylthioethyl-, ethylthiomethyl-, ethylthioethyl-, chlorid-, chlormethyl- vinyl-, phenyl-, benzyl-, methyl-, ethyl- , propyl-, isopropyl- and tert-butyl or cyclohexyl groups.

9. Conjugates of nucleic acids with two or more pH-responsive transfection enhancer elements (TEE's) as claimed in any of claims 1 to 8 wherein said hydrophobic element comprises linear, branched or cyclic chains with a minimum chain length of 6 elements.

10. Conjugates of nucleic acids with two or more pH-responsive transfection enhancer elements (TEE's) as claimed in any of claims 1 to 9 wherein said hydrophobic element comprises more than 6 and up to 40 chain elements.

11. Conjugates of nucleic acids with two or more pH-responsive transfection enhancer elements (TEE's) as claimed in any of claims 1 to 10 wherein said hydrophobic element comprises between 6 and 20 chain elements.

12. Conjugates of nucleic acids with two or more pH-responsive transfection enhancer elements (TEE's) as claimed in any of claims 1 to 10 wherein said hydrophobic element comprises between 20 and 40 chain elements.

13. Conjugates of nucleic acids with two or more pH-responsive transfection enhancer elements (TEE's) as claimed in any of claims 1 to 12 wherein the chain elements of said hydrophobic element are carbon atoms.

14. Conjugates of nucleic acids with two or more pH-responsive transfection enhancer elements (TEE's) as claimed in any of claims 1 to 13 wherein said hydrophobic element can be saturated or may contain unsaturated bonds.

**15.** Conjugates of nucleic acids with two or more pH-responsive transfection enhancer elements (TEE's) as claimed in any of claims 1 to 14 wherein said hydrophobic element may be substituted.

**16.** Conjugates of nucleic acids with two or more pH-responsive transfection enhancer elements (TEE's) as claimed in any of claims 1 to 15 wherein the branching of the main chain of said hydrophobic element comprises one or more rather small building blocks such as methyl-, ethyl-, propyl-, isopropyl-, methoxy-, ethoxy-, methoxymethyl-, ethoxymethyl-, methoxyethyl-, ethoxyethyl- and vinyl- or halogen groups or mixtures thereof.

**17.** Conjugates of nucleic acids with two or more pH-responsive transfection enhancer elements (TEE's) as claimed in any of claims 1 to 16 wherein said hydrophobic element derives from sterols.

**18.** Conjugates of nucleic acids with two or more pH-responsive transfection enhancer elements (TEE's) as claimed in claim 17
wherein said sterols are further substituted with substituents selected from the group comprising methyl-, ethyl-, propyl-, isopropyl-, methoxy-, ethoxy-, methoxymethyl-, ethoxymethyl-, methoxyethyl-, ethoxyethyl- and vinyl-, halogen- or hydroxyl-groups or mixtures thereof.

**19.** Conjugates of nucleic acids with two or more pH-responsive transfection enhancer elements (TEE's) as claimed in any of claims 1 to 18 wherein said hydrophobic element comprises one or more heteroatoms or chemical linking groups, selected from the group comprising -O-, -S-, -N(H)C(O)-, -C(O)O-, -OC(O)N(H)-, -C(O)-, - C(O)-N(H)-, -N (H)-C(O)-O-, -CH=N-, -O-C(O)-, -N=CH- and/or -S-S-, amino acids or derivatives thereof, $\alpha$-hydroxyacids or $\beta$-hydroxy acids.

**20.** Conjugates of nucleic acids with two or more pH-responsive transfection enhancer elements (TEE's) as claimed in any of claims 1 to 19 wherein said pH-responsive transfection enhancer elements (TEE's) have a difference in logD (4.0)-logD(7.4) that is greater than 1 logD unit.

**21.** Conjugates of nucleic acids with two or more pH-responsive transfection enhancer elements (TEE's) as claimed in any of claims 1 to 20 wherein said pH-responsive transfection enhancer elements (TEE's) have a logD at pH 7.4 of between -2 and 10.

**22.** Conjugates of nucleic acids with two or more pH-responsive transfection enhancer elements (TEE's) as claimed in any of claims 1 to 20 wherein polymer fragments or monomers of said nucleic acids are modified with one or more TEE's and the sum of logD at pH 7.4 (polymer fragment) and logD at pH 7.4 of said pH-responsive transfection enhancer elements (TEE's) is higher than -10 and preferred higher than -5.

**23.** Conjugates of nucleic acids with two or more pH-responsive transfection enhancer elements (TEE's) as claimed in any of claims 1 to 22 wherein the logD at pH 4 of said pH-responsive transfection enhancer elements (TEE's) exceeds 0.

**24.** Conjugates of nucleic acids with two or more pH-responsive transfection enhancer elements (TEE's) as claimed in any of claims 1 to 23 wherein said pH-responsive transfection enhancer elements (TEE's) comprise more than one pH-responsive hydrophilic element.

**25.** Conjugates of nucleic acids with two or more pH-responsive transfection enhancer elements (TEE's) as claimed in any of claims 1 to 24 wherein said pH-responsive transfection enhancer elements (TEE's) are grafted onto said nucleic acid via covalent chemical bonds.

**26.** Conjugates of nucleic acids with two or more pH-responsive transfection enhancer elements (TEE's) as claimed in claim 25 wherein said pH-responsive transfection enhancer elements (TEE's) may be grafted onto said nucleic acid at positions including the 2'O- or 2'S-positions of the nucleosides, the nucleobases, the internucleoside linkages, e.g. the phosphate backbone or modifications thereof, the peptide backbone in peptide nucleic acids, the heterocyclic backbone in morpholino nucleic acids and the like or the graft may also be attached to the phosphoribose or phosphodexyribose backbone, replacing the former nucleobases at Cl.

**27.** Conjugates of nucleic acids with two or more pH-responsive transfection enhancer elements (TEE's) as claimed in any of claims 25 to 26 wherein all or no more than 2/3 or no more than 1/3 of all nucleobases of said nucleic acid are modified with said pH-responsive transfection enhancer elements (TEE's).

**28.** Conjugates of nucleic acids with two or more pH-responsive transfection enhancer elements (TEE's) as claimed in any of claims 25 to 27 wherein only nucleobases at the flanks of said nucleic acids are modified with pH-responsive transfection enhancer elements (TEE's)leading to gapmer structures.

**29.** Conjugates of nucleic acids with two or more pH-responsive transfection enhancer elements (TEE's) as claimed in any of claims 25 to 28 wherein said nucleic acid comprise polymeric or oligomeric extensions and wherein said polymer backbone structure is not a phosphoribose and said extensions carry one or more TEE's.

**30.** Conjugates of nucleic acids with two or more pH-responsive transfection enhancer elements (TEE's) as claimed in claims 29 wherein said extension represents peptides or polyesters forming oligomeric or multimeric forms of TEE's including TEE's linked to peptides, $\alpha$- or $\beta$-amino acids or $\alpha$- or $\beta$-hydroxyacids.

**31.** Conjugates of nucleic acids with two or more pH-responsive transfection enhancer elements (TEE's) as claimed in any of claims 25 to 30 wherein said pH-responsive transfection enhancer elements (TEE's) are alkylcarboxylic acids with 6 or more carbon atoms, preferred between 8 to 16 carbon atoms.

**32.** Conjugates of nucleic acids with two or more pH-responsive transfection enhancer elements (TEE's) as claimed in any of claims 25 to 30 wherein said pH-responsive transfection enhancer elements (TEE's) are sterol-based.

**33.** Conjugates of nucleic acids with two or more pH-responsive transfection enhancer elements (TEE's) as claimed in any of claims 1 to 24 wherein one or more of said pH-responsive transfection enhancer elements are grafted onto polycationic elements that form a precipitate, a complex controlled in size or a stoichiometric associate with said nucleic acids.

**34.** Conjugates of nucleic acids with two or more pH-responsive transfection enhancer elements (TEE's) as claimed in claim 33 wherein said polycationic elements are polyamines selected from the group comprising polyethyleneimine, spermine, thermine, spermidine, putrescine, polylysine or polyarginine and derivatives thereof.

**35.** Conjugates of nucleic acids with two or more pH-responsive transfection enhancer elements (TEE's) as claimed in claim 33 wherein said polycationic elements are lipopolyamines selected from the group comprising cholesteryl polyamine carbamates, DOSPER, DOGS or DOSPA.

**36.** Conjugates of nucleic acids with two or more pH-responsive transfection enhancer elements (TEE's) as claimed in any of claims 1 to 35 wherein said nucleic acids are oligonucleotides, polynucleotides or DNA plasmids.

**37.** Conjugates of nucleic acids with two or more pH-responsive transfection enhancer elements (TEE's) as claimed in claim 36 wherein said nucleic acids are capable of being transcribed in a vertebrate cell into one or more RNAs, said RNAs being mRNAs, shRNAs, miRNAs or ribozymes, said mRNAs coding for one or more proteins or polypeptides.

**38.** Conjugates of nucleic acids with two or more pH-responsive transfection enhancer elements (TEE's) as claimed in claim 37 wherein said nucleic acid is a circular DNA plasmid, a linear DNA construct or an mRNA.

**39.** Conjugates of nucleic acids with two or more pH-responsive transfection enhancer elements (TEE's) as claimed in claim 36 wherein said nucleic acid is an oligonucleotide.

**40.** Conjugates of nucleic acids with two or more pH-responsive transfection enhancer elements (TEE's) as claimed in claim 39 wherein said oligonucleotide is a decoy oligonucleotide, an antisense oligonucleotide, a siRNA, an agent influencing transcription, an agent influencing splicing, Ribozymes, DNAzymes or Aptamers.

**41.** Conjugates of nucleic acids with two or more pH-responsive transfection enhancer elements (TEE's) as claimed in claim 39 or claim 40 wherein said oligonucleotides comprise naturally occurring or modified nucleosides such as DNA, RNA, locked nucleic acids (LNA's), 2'O-methyl RNA (2'Ome), 2' O-methoxyethyl RNA (2'MOE) in their phosphate or phosphothioate forms or Morpholinos or peptide nucleic acids (PNA's).

**42.** Conjugates of nucleic acids with two or more pH-responsive transfection enhancer elements (TEE's) as claimed in claim 39, claim 40 or claim 41 wherein said oligonucleotide is an antisense oligonucleotide of 8 to 50 basepairs length.

**43.** Conjugates of nucleic acids with two or more pH-responsive transfection enhancer elements (TEE's) as claimed in claim 39, claim 40 or claim 41 wherein said oligonucleotide is a siRNA of 15 to 30 basepairs length.

**44.** Conjugates of nucleic acids with two or more pH-responsive transfection enhancer elements (TEE's) as claimed in claim 39, claim 40 or claim 41 wherein said oligonucleotide is a decoy oligonucleotide of 15 to 30 basepairs length.

**45.** Conjugates of nucleic acids with two or more pH-responsive transfection enhancer elements (TEE's) as claimed in claim 39, claim 40 or claim 41 wherein said oligonucleotide is an agent influencing the transcription of 15 to 30 basepairs length.

**46.** Conjugates of nucleic acids with two or more pH-responsive transfection enhancer elements (TEE's) as claimed in claim 39, claim 40 or claim 41 wherein said oligonucleotide is a DNAzyme of 25 to 50 basepairs length.

**47.** Conjugates of nucleic acids with two or more pH-responsive transfection enhancer elements (TEE's) as claimed in claim 39, claim 40 or claim 41 wherein said oligonucleotide is a Ribozyme of 25 to 50 basepairs length.

**48.** Conjugates of nucleic acids with two or more pH-responsive transfection enhancer elements (TEE's) as claimed in claim 39, claim 40 or claim 41 wherein said oligonucleotide is a Aptamer of 15 to 60 basepairs length.

**49.** A pharmaceutical composition comprising conjugates of nucleic acids with two or more pH-responsive transfection enhancer elements (TEE's) as claimed in any of claims 1 to 48 and a pharmaceutically acceptable vehicle therefore.

**50.** Use of a pharmaceutical composition as claimed in claim 49, for the treatment or prophylaxis of inflammatory, immune or autoimmune disorders and/or cancer of humans or non-human animals.

**51.** A method of treating a human or non-human animal by administering a pharmaceutical composition as claimed in any of claims 49 to 50, wherein said conjugates targeting specific organ or organs, tumours or sites of infection or inflammation.

**52.** Use of pH responsive transfection enhancer elements (TEE's) of the general formula I

Hydrophobic element - pH-responsive hydrophilic elements (I)

for the in vivo, in vitro or ex vivo transfection of polar solutes; wherein said pH-responsive hydrophilic elements comprises weak acids having a pka of between 2 and 6, preferred of between 3 and 5 or
is a zwitterionic structure comprising a combination of weak or strong acidic groups with weak bases having a pka of between 3 and 8, preferred of between 4.5 and 7.
and
wherein said hydrophobic element comprises linear, branched or cyclic chains with a minimum chain length of 6 elements.

**53.** Use of pH responsive transfection enhancer elements (TEE's)as claimed in claim 52 wherein said pH-responsive hydrophilic element is located distal from the link between said polar solute and TEE.

**54.** Use of pH responsive transfection enhancer elements (TEE's)as claimed in claim 52 wherein said pH-responsive hydrophilic element is located central within the TEE.

**55.** Use of pH responsive transfection enhancer elements (TEE's) as claimed in any of claim 52 to 54 wherein said pH-responsive hydrophilic element may further be substituted with structural elements, selected from the group comprising hydroxymethyl-, hydroxyethyl-, methoxymethyl-, methoxyethyl-, ethoxymethyl-, ethoxyethyl-, thiomethyl-, thioethyl-, methylthiomethyl-, methylthioethyl-, ethylthiomethyl-, ethylthioethyl-, chlorid-, chlormethyl- vinyl-, phenyl-, benzyl-, methyl-, ethyl- , propyl-, isopropyl- and tert-butyl or cyclohexyl groups.

**56.** Use of pH responsive transfection enhancer elements (TEE's) as claimed in any of claim 52 to 55 wherein said hydrophobic element comprises more than 6 and up to 40 chain elements.

**57.** Use of pH responsive transfection enhancer elements (TEE's) as claimed in any of claims 52 to 56 wherein the chain elements of said hydrophobic element are carbon atoms.

**58.** Use of pH responsive transfection enhancer elements (TEE's) as claimed in any of claims 52 to 57 wherein said hydrophobic element can be saturated or may contain unsaturated bonds.

**59.** Use of pH responsive transfection enhancer elements (TEE's) as claimed in any of claims 52 to 58 wherein said hydrophobic element may be substituted.

**60.** Use of pH responsive transfection enhancer elements (TEE's) as claimed in any of claims 52 to 59 wherein the branching of the main chain of said hydrophobic element comprises one or more rather small building blocks such as methyl-, ethyl-, propyl-, isopropyl-, methoxy-, ethoxy-, methoxymethyl-, ethoxymethyl-, methoxyethyl-, ethoxyethyl- and vinyl- or halogen groups or mixtures thereof.

**61.** Use of pH responsive transfection enhancer elements (TEE's) as claimed in any of claims 52 to 60 wherein said hydrophobic element derives from sterols.

**62.** Use of pH responsive transfection enhancer elements (TEE's) as claimed in claim 61 wherein said sterols are further substituted with substituents selected from the group comprising methyl-, ethyl-, propyl-, isopropyl-, methoxy-, ethoxy-, methoxymethyl-, ethoxymethyl-, methoxyethyl-, ethoxyethyl- and vinyl-, halogen- or hydroxyl-groups or mixtures thereof.

**63.** Use of pH responsive transfection enhancer elements (TEE's) as claimed in any of claims 52 to 62 wherein said hydrophobic element comprises one or more heteroatoms or chemical linking groups, selected from the comprising -O-, -S-, -N(H)C(O)-, -C(O)O-, - OC(O)N(H)-, -C(O)-, -C(O)-N(H)-, -N(H)-C(O)-O-, -CH=N-, -O-C(O)-, - N=CH- and/or -S-S-, amino acids or derivatives thereof, α-hydroxyacids or β-hydroxy acids.

**64.** Use of pH responsive transfection enhancer elements (TEE's) as claimed in any of claims 52 to 63 wherein said pH responsive transfection enhancer elements (TEE's) have a difference logD(4.0)-logD(7.4) that is greater than 1 logD unit.

**65.** Use of pH responsive transfection enhancer elements (TEE's) as claimed in any of claims 52 to 63 wherein said pH-responsive transfection enhancer elements (TEE's) has a logD at pH 7.4 of between -2 and 10.

**66.** Use of pH responsive transfection enhancer elements (TEE's) as claimed in any of claims 52 to 63 wherein said polar solutes or polymer fragments or monomers thereof are modified with one or more TEE's and the sum of logD at pH 7.4 of said polar solute or said polymer fragment and logD at pH 7.4 of said pH-responsive transfection enhancer elements (TEE's) is higher than -10 and preferred higher than -5.

**67.** Use of pH responsive transfection enhancer elements (TEE's) as claimed in any of claims 52 to 66 wherein the logD at pH 4 of said pH-responsive transfection enhancer elements (TEE's) exceeds 0.

**68.** Use of pH responsive transfection enhancer elements (TEE's) as claimed in any of claims 52 to 67 wherein said pH-responsive transfection enhancer elements (TEE's) comprise more than one pH-responsive hydrophilic element.

**69.** Use of pH responsive transfection enhancer elements (TEE's) as claimed in any of claims 52 to 68 wherein said polar solutes are selected from the group comprising small molecules, proteins, peptides, carbohydrates or nucleic acids.

**70.** Use of pH responsive transfection enhancer elements (TEE's) as claimed in any of claims 52 to 69 wherein said one or more transfection enhancer elements (TEE's) may be conjugated to said polar solutes by chemical bonds, physical attraction or by other interactions.

**71.** Use of pH responsive transfection enhancer elements (TEE's) as claimed in any of claims 52 to 69 wherein said transfection enhancer elements (TEE's) are conjugated to carrier systems sequestering said polar solutes.

**72.** Conjugates of nucleic acids with one pH-responsive transfection enhancer element (TEE) with the general structure (I) Hydrophobic element - pH-responsive hydrophilic elements (I) wherein said conjugates comprise a TEE other than a saturated, straight chain 1-carboxylic acid.

Figure 1:

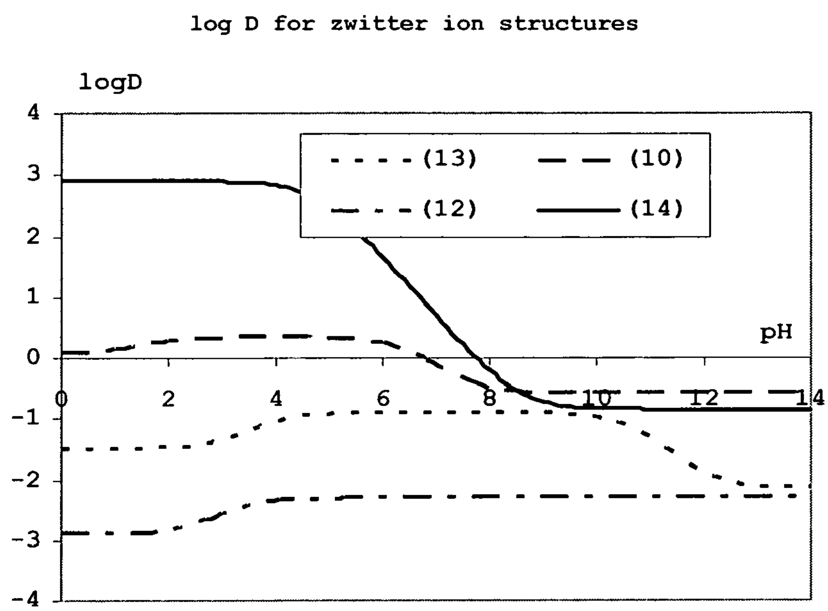
log D for zwitter ion structures
logD
4
3
2
1
0
-1
-2
-3
-4
0
2
4
6
8
10
12
14
pH
(13)
(10)
(12)
(14)

Figure 2:

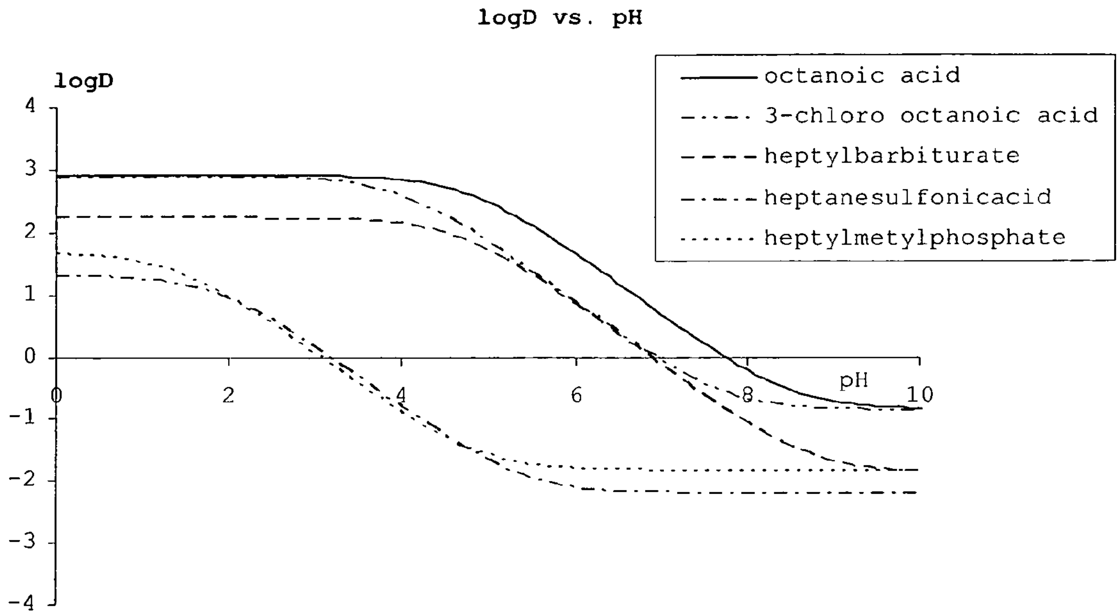
logD vs. pH
logD
4
3
2
1
0
-1
-2
-3
-4
0
2
4
6
8
10
pH
octanoic acid
3-chloro octanoic acid
heptylbarbiturate
heptanesulfonicacid
heptylmetylphosphate

Figure 3:

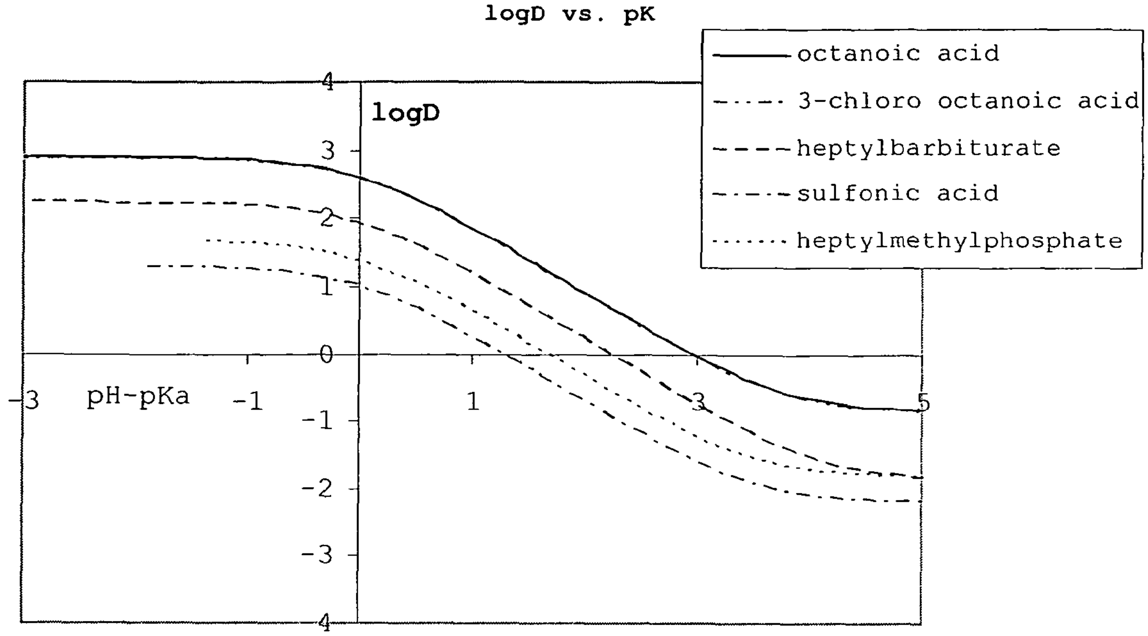
logD vs. pK
octanoic acid
3-chloro octanoic acid
heptylbarbiturate
sulfonic acid
heptylmethylphosphate
logD
pH-pKa
4
3
2
1
0
-1
-2
-3
-4
-3
-1
1
3
5

Figure 4:

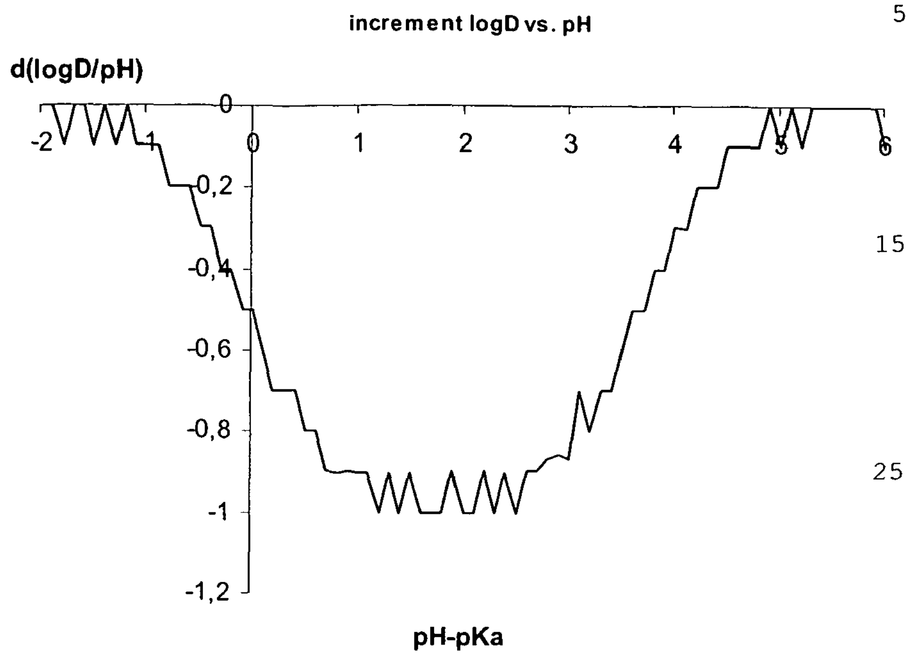
increment logD vs. pH
d(logD/pH)
0
-0,2
-0,4
-0,6
-0,8
-1
-1,2
-2
-1
0
1
2
3
4
5
6
pH-pKa

Figure 5:

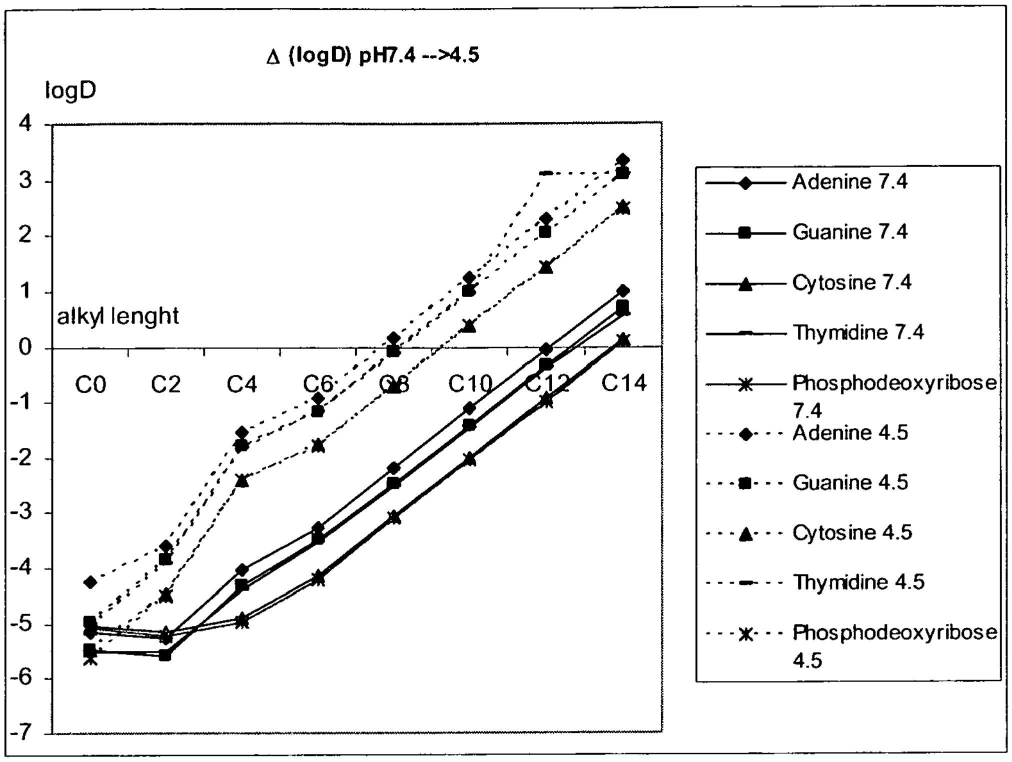
Δ (logD) pH7.4 -->4.5
logD
4
3
2
1
0
-1
-2
-3
-4
-5
-6
-7
alkyl lenght
C0
C2
C4
C6
C8
C10
C12
C14
Adenine 7.4
Guanine 7.4
Cytosine 7.4
Thymidine 7.4
Phosphodeoxyribose 7.4
Adenine 4.5
Guanine 4.5
Cytosine 4.5
Thymidine 4.5
Phosphodeoxyribose 4.5

Figure 6:

(A)

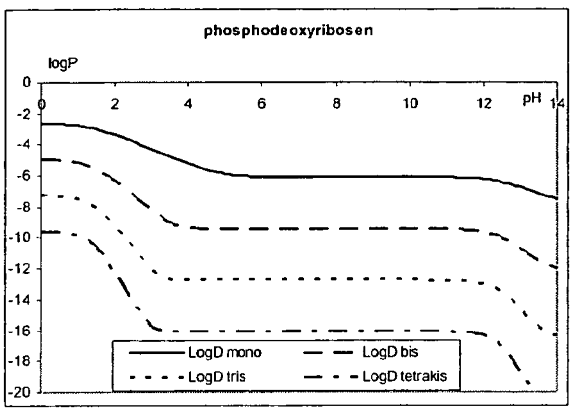
phosphodeoxyribosen
logP
pH
LogD mono
LogD bis
LogD tris
LogD tetrakis

(B)

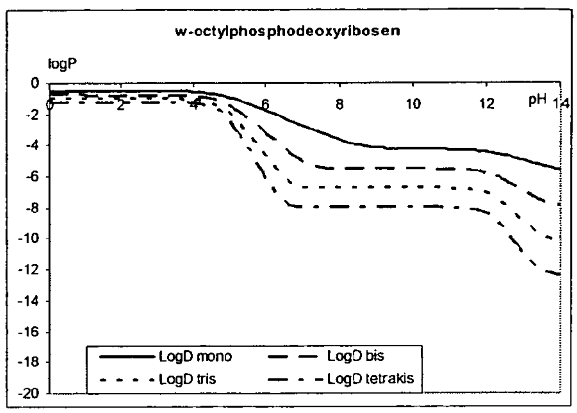
w-octylphosphodeoxyribosen
logP
pH
LogD mono
LogD bis
LogD tris
LogD tetrakis

European Patent Office

# PARTIAL EUROPEAN SEARCH REPORT

which under Rule 63 of the European Patent Convention shall be considered, for the purposes of subsequent proceedings, as the European search report

Application Number EP 06 07 7234

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 97/40854 A (ANTIVIRALS INC [US]) 6 November 1997 (1997-11-06)<br>* page 2, line 31 - page 3, line 16 *<br>* page 38 - page 40; example 6C *<br>* page 44 - page 46; example 10B *<br>* claims *<br>----- | 1-5,13, 24,25, 36, 38-41, 52,69, 70,72 | INV. A61K47/48 |
| X | WO 98/32467 A (ANTIVIRALS INC [US]; UNIV NEBRASKA [US]) 30 July 1998 (1998-07-30)<br>* page 1, lines 29,30 *<br>* page 2, lines 23-33 *<br>* page 3, lines 1-11 *<br>* example 11 *<br>* claims *<br>-----<br>-/-- | 1-5,13, 24,25, 36, 38-41, 49-52, 69,70,72 | |

TECHNICAL FIELDS SEARCHED (IPC)

A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 12 October 2007 | Villard, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

7

EPO FORM 1503 03.82 (P04E07)

European Patent Office

# PARTIAL EUROPEAN SEARCH REPORT

Application Number
EP 06 07 7234

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HENRY SCOTT M ET AL: "pH-responsive poly(styrene-alt-maleic anhydride) alkylamide copolymers for intracellular drug delivery" BIOMACROMOLECULES, vol. 7, no. 8, August 2006 (2006-08), pages 2407-2414, XP002454257 ISSN: 1525-7797 * abstract * * page 2407, column 1, paragraph 1 * * figure 1 * ----- | 1-5,9, 10,13, 24, 36-41, 49-52, 69,70,72 | |
| X | CHEUNG CHARLES Y ET AL: "A pH-sensitive polymer that enhances cationic lipid-mediated gene transfer" BIOCONJUGATE CHEMISTRY, vol. 12, no. 6, November 2001 (2001-11), pages 906-910, XP002454175 ISSN: 1043-1802 * abstract * * page 909, column 2, last paragraph * ----- | 1-5,9, 10,13, 24, 36-41, 49-52, 69-72 | TECHNICAL FIELDS SEARCHED (IPC) |
| X | FATTAL E ET AL: ""Smart" delivery of antisense oligonucleotides by anionic pH-sensitive liposomes" ADVANCED DRUG DELIVERY REVIEWS 23 APR 2004 NETHERLANDS, vol. 56, no. 7, 23 April 2004 (2004-04-23), pages 931-946, XP002454176 ISSN: 0169-409X * abstract * * pages 937-938, paragraph 4.3 * * pages 940-943, paragraphs 6,7 * ----- | 1-5, 8-10,13, 17,24, 36, 38-41, 49-52, 69-72 | |

-/--

7

EPO FORM 1503 03.82 (P04C10)

European Patent Office

# PARTIAL EUROPEAN SEARCH REPORT

Application Number
EP 06 07 7234

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X | POTIER P ET AL: "Synthesis and hybridization properties of oligonucleotides containing polyamines at the C-2 position of purines: a pre-synthetic approach for the incorporation of spermine into oligodeoxynucleotides containing 2-(4,9,13-triazatridecyl)-2'-deoxyguanosine." CHEMISTRY (WEINHEIM AN DER BERGSTRASSE, GERMANY) 17 NOV 2000, vol. 6, no. 22, 17 November 2000 (2000-11-17), pages 4188-4194, XP002454258 ISSN: 0947-6539 * abstract * * page 4192, column 1, paragraph 1 * ----- | 1-3,13, 24,25, 36, 38-41, 49-51,72 | |
| X | REMY J-S ET AL: "GENE TRANSFER WITH A SERIES OF LIPOPHILIC DNA-BINDING MOLECULES" BIOCONJUGATE CHEMISTRY, ACS, WASHINGTON, DC, US, vol. 5, no. 6, 1 November 1994 (1994-11-01), pages 647-654, XP000484178 ISSN: 1043-1802 * abstract * * figure 1 * ----- | 1-3, 8-10,13, 24,33, 36-41, 49-52, 69-72 | TECHNICAL FIELDS SEARCHED (IPC) |
| A | WO 96/07392 A (HYBRIDON INC [US]; IYER RADHAKRISHNAN P [US]; YU DONG [US]; AGRAWAL SU) 14 March 1996 (1996-03-14) * page 19, lines 5-26 * * page 7, lines 4-10 * ----- | | |
| A | US 5 643 889 A (SUHADOLNIK ROBERT J [US] ET AL) 1 July 1997 (1997-07-01) * claims * ----- | | |

7

EPO FORM 1503 03.82 (P04C10)

European Patent Office

**INCOMPLETE SEARCH SHEET C**

Application Number
EP 06 07 7234

Although claims 50-52,69-72 are directed to a method of treatment of the human/animal body (Article 52(4) EPC), the search has been carried out and based on the alleged effects of the compound/composition.

-----

European Patent Office

Application Number

EP 06 07 7234

## CLAIMS INCURRING FEES

The present European patent application comprised at the time of filing more than ten claims.

- [ ] Only part of the claims have been paid within the prescribed time limit. The present European search report has been drawn up for the first ten claims and for those claims for which claims fees have been paid, namely claim(s):

- [ ] No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [X] None of the further search fees have been paid within the fixed time limit. The present European search report has been drawn up for those parts of the European patent application which relate to the invention first mentioned in the claims, namely claims:

  see annex

- [ ] The present supplementary European search report has been drawn up for those parts of the European patent application which relate to the invention first mentioned in the claims (Rule 164 (1) EPC).

European Patent Office

LACK OF UNITY OF INVENTION
SHEET B

Application Number
EP 06 07 7234

The Search Division considers that the present European patent application does not comply with the requirements of unity of invention and relates to several inventions or groups of inventions, namely:

1. claims: 31; 1-5,8-10,13,17,24,25,32,33,36-41,49-52,69-72 (in part)

Conjugate of a nucleic acid with two or more pH-responsive transfection enhancer elements with the general structure hydrophobic element - pH-responsive hydrophobic element, wherein the pH-responsive hydrophilic element comprises a carboxylic acid group; pharmaceutical composition thereof and use of this pharmaceutical composition for the treatment or prophylaxis of inflammatory, immune or autoimmune disorders and/or cancer; use of the above mentioned transfection enhancer element for the in-vivo, in-vitro or ex-vivo transfection of polar solutes.

---

2. claims: 1-5,8-10,13,17,24,25,32,33,36-41,49-52,69-72 (in part)

Conjugate of a nucleic acid with two or more pH-responsive transfection enhancer elements with the general structure hydrophobic element - pH-responsive hydrophobic element, wherein the pH-responsive hydrophilic element comprises a barbituric acid group; pharmaceutical composition thereof and uses of this pharmaceutical composition for the treatment or prophylaxis of inflammatory, immune or autoimmune disorders and/or cancer; use of the above mentioned transfection enhancer element for the in-vivo, in-vitro or ex-vivo transfection of polar solutes.

---

3. claims: 1-5,8-10,13,17,24,25,32,33,36-41,49-52,69-72 (in part)

Conjugate of a nucleic acid with two or more pH-responsive transfection enhancer elements with the general structure hydrophobic element - pH-responsive hydrophobic element, wherein the pH-responsive hydrophilic element comprises a xanthine group; pharmaceutical composition thereof and uses of this pharmaceutical composition for the treatment or prophylaxis of inflammatory, immune or autoimmune disorders and/or cancer; use of the above mentioned transfection enhancer element for the in-vivo, in-vitro or ex-vivo transfection of polar solutes.

---

4. claims: 1-5,8-10,13,17,24,25,32,33,36-41,49-52,69-72 (in part)

European Patent Office

# LACK OF UNITY OF INVENTION SHEET B

Application Number
EP 06 07 7234

The Search Division considers that the present European patent application does not comply with the requirements of unity of invention and relates to several inventions or groups of inventions, namely:

Conjugate of a nucleic acid with two or more pH-responsive transfection enhancer elements with the general structure hydrophobic element - pH-responsive hydrophobic element, wherein the pH-responsive hydrophilic element comprises a pyrimidine or uracil group; pharmaceutical composition thereof and uses of this pharmaceutical composition for the treatment or prophylaxis of inflammatory, immune or autoimmune disorders and/or cancer; use of the above mentioned transfection enhancer element for the in-vivo, in-vitro or ex-vivo transfection of polar solutes.

---

5. claims: 6,7; 1-3,8-10,13,17,24,25,32,33,36-41,49-52,69-72 (in part)

Conjugate of a nucleic acid with two or more pH-responsive transfection enhancer elements with the general structure hydrophobic element - pH-responsive hydrophobic element, wherein the pH-responsive hydrophilic element is a zwitterionic structure comprising a combination of weak or strong acidic groups with weak bases having a pka of between 3 and 8, preferred of between 4.7 and 7; pharmaceutical composition thereof and uses of this pharmaceutical composition for the treatment or prophylaxis of inflammatory, immune or autoimmune disorders and/or cancer; use of the above mentioned transfection enhancer element for the in-vivo, in-vitro or ex-vivo transfection of polar solutes.

---

## ANNEX TO THE EUROPEAN SEARCH REPORT ON EUROPEAN PATENT APPLICATION NO. EP 06 07 7234

This annex lists the patent family members relating to the patent documents cited in the above-mentioned European search report.
The members are as contained in the European Patent Office EDP file on
The European Patent Office is in no way liable for these particulars which are merely given for the purpose of information.

12-10-2007

| Patent document cited in search report | Publication date | Patent family member(s) | Publication date |
|---|---|---|---|
| WO 9740854 A | 06-11-1997 | AU 729643 B2 | 08-02-2001 |
| | | AU 2929897 A | 19-11-1997 |
| | | CA 2252706 A1 | 06-11-1997 |
| | | EP 0966303 A2 | 29-12-1999 |
| | | JP 2000509394 T | 25-07-2000 |
| WO 9832467 A | 30-07-1998 | AT 291617 T | 15-04-2005 |
| | | AU 742521 B2 | 03-01-2002 |
| | | AU 5930598 A | 18-08-1998 |
| | | CA 2278924 A1 | 30-07-1998 |
| | | DE 69829469 D1 | 28-04-2005 |
| | | EP 0973886 A2 | 26-01-2000 |
| | | JP 2001509167 T | 10-07-2001 |
| | | US 6124271 A | 26-09-2000 |
| WO 9607392 A | 14-03-1996 | AT 202569 T | 15-07-2001 |
| | | AU 3675195 A | 27-03-1996 |
| | | CA 2199464 A1 | 14-03-1996 |
| | | DE 69521517 D1 | 02-08-2001 |
| | | DE 69521517 T2 | 18-04-2002 |
| | | EP 0779893 A2 | 25-06-1997 |
| | | JP 10505094 T | 19-05-1998 |
| US 5643889 A | 01-07-1997 | US 5550111 A | 27-08-1996 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4458066 A
- WO 06105361 A
- US 6320041 B
- US 4958013 A, Letsinger
- US 6153737 A, Manoharan
- US 6753423 B
- WO 9821322 A
- DE 19753182
- EP 1392341 A
- WO 9932619 A
- WO 02055693 A


### Non-patent literature cited in the description


- **LIPINSKI et al.** *Advanced Drug Delivery Reviews,* 1997, vol. 23, 3-25
- **MADDEN et al.** *Chem. Phys. Lipids,* 1990, vol. 53 (1), 37-46
- **HARRIGAN et al.** *Biochim. Biophys. Acta,* 1993, vol. 1149 (2), 329-338
- **CLERCS et al.** *Biochim. Biophys. Acta,* 1995, vol. 1240 (2), 257-265
- **NARANG et al.** *Meth. Enzymol,* 1979, vol. 68, 90-99
- **BROWN et al.** *Method Enzymol.,* 1979, vol. 68, 109-151
- **BEAUCAGE et al.** *Tetrahedron Lett.,* 1981, vol. 22, 1859-1862
- **MATTEUCCI et al.** *J. Am. Chem. Soc.,* 1981, vol. 103, 3185-3191
- **BOUSSIF et al.** *Proc. Natl. Acad. Sci.,* 1995, vol. 92 (16), 7297-7301
- **GODBEY et al.** *J. Control. Release,* 1999, vol. 60 (2-3), 149-160
- **JANES et al.** *Adv. Drug Deliv. Rev,* 2001, vol. 47 (1), 83-97
- **HU-LIESKOVAN et al.** *Cancer Res.,* 2005, vol. 65 (19), 8984-8992
- **KAUL et al.** *Pharm. Res,* 2005, vol. 22 (6), 951-961
- **SANO et al.** *Adv. Drug Deliv. Rev,* 2003, vol. 55 (12), 1651-1677
- **G. T. HERMANSON.** Bioconjugate Techniques. Academic Press, 1996
- **DEGRIP et al.** *Biochem. J.,* 1998, vol. 330, 667-674
- *Proc. Natl. Acad. Sci.,* 1989, vol. 86, 6553-6556
- **SOUTSCHEK et al.** *Nature,* 2004, vol. 432 (7014), 173-178
- **CHO-CHUNG et al.** *Curr. Opin. Mol. Ther,* 1999